# EUROPÄISCHE PATENTSCHRIFT

(11) **EP 4 327 760 B1**
(45) Veröffentlichungstag und Bekanntmachung des Hinweises auf die Patenterteilung: **01.10.2025**
(21) Anmeldenummer: 22192132.3
(22) Anmeldetag: 25.08.2022
(51) Int. Cl.: A61B 17/22, A61B 17/00

(54) **DRUCKWELLENGERÄT MIT DOPPELVENTILEINRICHTUNG**
PRESSURE WAVE DEVICE WITH DOUBLE VALVE DEVICE
APPAREIL À ONDES DE CHOC POURVU DE DISPOSITIF À DOUBLE SOUPAPE

(43) Veröffentlichungstag der Anmeldung: 28.02.2024
(73) Patentinhaber: STORZ MEDICAL AG, 8274 Tägerwilen (CH)
(72) Erfinder: STORZ, Rafael, 8280 Kreuzlingen (CH); BELAU, Markus, 78467 Konstanz (DE); KÜHL, Arvid, 8274 Tägerwilen (CH); HONSELL, Lukas, 78479 Reichenau (DE); GREMLICH, Felix, 8280 Kreuzlingen (CH); GLENZER, Thomas, 8280 Kreuzlingen (CH)
(74) Vertreter: Szynka Smorodin Patentanwälte Partnerschaft mbB

(56) Entgegenhaltungen:
- EP-A1- 2 529 679
- EP-B1- 2 213 273
- US-A1- 2009 326 425
- US-A1- 2011 275 965
- US-A1- 2014 350 438

## Beschreibung

Die Erfindung bezieht sich auf ein Gerät zur Behandlung des menschlichen oder tierischen Körpers mit mechanischen Druckwellen, welche durch Aufschlagen eines beschleunigten Projektils auf einen Applikator erzeugt werden.

Geräte dieses Typs sind seit einiger Zeit bekannt und zunehmend im Einsatz. Zur Behandlung des (menschlichen oder tierischen) Patienten dienen mechanische Druckwellen, die durch Auflegen eines Applikators auf den Körper des Patienten eingekoppelt werden und durch eine Kollision eines beschleunigten Projektils mit dem Applikator erzeugt werden. Dabei muss der Applikator nicht zwingend einstückig sein, sondern kann auch aus einer Mehrzahl verschiedener Teile oder Materialien zusammengesetzt sein.

Eine in der Praxis bewährte und vielfach beschriebene Technik zur Beschleunigung des Projektils ist pneumatisch. Dabei wird durch Beaufschlagung eines Volumens auf einer Seite des entlang einer Bewegungsstrecke, z. B. in einem Rohrstück, beweglichen Projektils ein pneumatischer Überdruck eingekoppelt.

Im Stand der Technik wird hierfür ein Schaltventil benutzt, das an eine Pneumatikversorgung, insbesondere einen Kompressor mit regelbarem Ausgangsdruck, angeschlossen ist und dessen Puls das Projektil von einem zu dem Applikator distalen Ende der Bewegungsstrecke aus hin zu dem Applikator beschleunigt. Die pneumatische Beaufschlagung wird bei Erreichen des proximalen Endes der Bewegungsstrecke ausgeschaltet, also mit dem Aufschlagen auf dem Applikator.

Die Rückbewegung erfolgt im Stand der Technik mithilfe einer Gegendruckkammer, also eines Speichervolumens, in das das zu dem Applikator hin bewegte Projektil gewissermaßen die vor ihm befindliche Luft verdrängt, womit es dieses Speichervolumen quasi aufpumpt.

In der vorveröffentlichten, allerdings wegen mangelnder Ausführbarkeit im Einspruchsbeschwerdeverfahren widerrufenen EP 2 181 730 B1 wird neben einer nicht näher ausgeführten Steuerung der Öffnungszeit des Schaltventils für die Beschleunigung auch eine gezielte Druckbegrenzung in dieser Gegendruckkammer diskutiert. Außerdem erwähnt dieses Dokument den Einsatz eines zweiten Schaltventils für eine Rückführung des Projektils in die distale Ausgangslage nach der Beaufschlagung durch das erste Schaltventil.

Die US 2014/350438 A1 beschreibt ein ballistisches Druckwellengerät nach dem Oberbegriff des Anspruchs 1, das zur Erzeugung besonders intensiver Wellen zur Zerkleinerung von Körperkonkrementen ausgelegt ist. Zur Rückführung des Projektils wird unter anderem eine pneumatische Lösung mit einem zweiten Ventil vorgeschlagen.

Die US 2009/326425 A1 beschreibt ein ballistisches Druckwellengerät, dessen pneumatisches System dazu ausgelegt ist, dass in Folge eines Beschleunigungsvorgangs des Projektils mindestens zwei Kollisionen stattfinden.

Die DE 20 2010 009 899 U1 beschreibt ein elektromagnetisches Druckwellengerät, das zur Rückführung des Projektils und Vermeidung von Schäden einer Rückstellfeder einen zweiten Elektromagneten vorschlägt.

Der vorliegenden Erfindung liegt die Aufgabe zugrunde, auf dieser Grundlage ein hinsichtlich der Hin- und Rückbewegung des Projektils verbessertes Gerät des beschriebenen Typs mit pneumatischer Einrichtung zur Projektilbewegung anzugeben.

Zur Lösung dieser Aufgabe wird das Gerät gemäß Anspruch 1 vorgeschlagen. Bevorzugte Ausgestaltungen sind Gegenstand der abhängigen Ansprüche.

Dementsprechend weist das erfindungsgemäße Gerät als Teil seiner pneumatischen Einrichtung eine Doppelventileinrichtung zur Beaufschlagung des Projektils in beide Richtungen, also zu dem Applikator hin und umgekehrt von ihm weg in der Rückrichtung, auf, also z. B. die Kombination eines ersten und eines zweiten Ventils. Dies erfolgt typischerweise mehrfach und iterativ in einer Folge. Die Zeitphasen, in denen das Projektil so pneumatisch beaufschlagt wird, dass es sich in der Hinrichtung bewegt, also z. B. die Einschaltphase eines ersten Ventils, wird im Folgenden als erste Einschaltzeit bezeichnet und umgekehrt als zweite Einschaltzeit eine Zeitphase einer umgekehrten Beaufschlagung des Projektils. Erfindungsgemäß soll das Gerät dazu ausgelegt sein (also insbesondere eine darin vorhandene Steuereinrichtung so ausgelegt sein), dass eine zweite Einschaltzeit erst beginnt, nachdem die erste Einschaltzeit beendet und eine Abstandszeit abgelaufen ist. Es gibt also zwischen den beiden Ventilöffnungszeiten eine von Null verschiedene Abstandszeit.

Das Gleiche gilt (alternativ oder zusätzlich) in umgekehrter Reihenfolge, nämlich hinsichtlich einer Abstandszeit nach dem Ende einer zweiten Einschaltzeit und vor dem Beginn einer ersten Einschaltzeit.

Z. B. kann die erste Einschaltzeit deutlich vor dem Aufschlagen des Projektils auf dem Applikator beendet werden und die zweite Einschaltzeit zum Beispiel unmittelbar nach dem Aufschlagen beginnen. Dann wird nicht die gesamte verfügbare Zeit zwischen dem Bewegungsstart des Projektils und dem Aufschlagen auf dem Applikator für die Beschleunigung bei anstehendem beschleunigenden Druck genutzt, sondern nur ein erster Teil davon. Z. B. könnte damit die Aufschlaggeschwindigkeit des Projektils verringert werden, ohne den beschleunigenden pneumatischen Druck absenken zu müssen. Es kann z. B. gewünscht sein, diesen für eine möglichst schnelle Rückführung (mithilfe der zweiten Einschaltzeit) auf einem höheren Wert zu lassen als für die Beschleunigung in Hinrichtung momentan erforderlich.

Folglich kann in solcher Weise die Aufschlaggeschwindigkeit durch die Steuerung gesteuert werden, und zwar auch unabhängig von einer Druckveränderung (z. B. bei gleichbleibendem Druck).

Es kann von Vorteil sein, ohne Verringerung des pneumatischen Drucks trotzdem die Aufschlaggeschwindigkeit des Projektils bei der Kollision reduzieren zu können. Z. B. kann einerseits eine besonders schnelle Rückführung des Projektils (durch die zweite Ventilöffnungszeit) und damit eine relativ hohe Betriebsfrequenz erwünscht sein, wobei gleichzeitig aus therapeutischen Gründen oder wegen Schmerzempfindlichkeit des Patienten keine allzu hohen Intensitäten (also Aufschlaggeschwindigkeiten) beabsichtigt sind. Oder es kann gewünscht sein, kurz zuvor oder danach wieder mit einer höheren Aufschlaggeschwindigkeit zu arbeiten oder aus anderen Gründen den Druck nicht abzusenken.

Außerdem kann die Abstandszeit auch ganz oder teilweise zeitlich nach der Kollision liegen. Dann kann durch ein verzögertes Einsetzen der zweiten Einschaltzeit nach der Kollision z. B. eine zu schnelle Rückbewegung oder eine zu heftige Geschwindigkeit am Ende der Rückbewegung verhindert werden, ohne den beschleunigenden Druck (für die Hinrichtung) absenken zu müssen. In diesem Zusammenhang ist auch zu berücksichtigen, dass die Kollision selbst nach den Gesetzen der Impulserhaltung schon eine gewisse Beschleunigung des Projektils in der Rückrichtung bewirkt.

Natürlich lassen sich beide Aspekte kombinieren, nämlich ein Teil der Abstandszeit vor und ein anderer Teil der Abstandzeit nach der Kollision.

Bislang wurde in den Beispielen die Abstandszeit nach einer (an die Abstandszeit angrenzenden) ersten Ventilöffnungszeit und vor einer folgenden zweiten Ventilöffnungszeit betrachtet. Es kann aber zusätzlich oder alternativ auch die Variation der Abstandszeit zwischen einer vorhergehenden (angrenzenden) zweiten und einer dann folgenden ersten Ventilöffnungszeit variiert werden. Z. B. kann eine solche Abstandszeit durch ein verzögertes (und hinsichtlich der Verzögerung variiertes) Einsetzen der ersten Ventilöffnungszeit bei der Beschleunigung in Hinrichtung relevant sein. Die Projektilbewegung könnte also zunächst ausschließlich durch eine "Reflexion" am distalen Ende der Bewegungsstrecke beginnen, also durch einen Restimpuls des Projektils nach einer Kollision mit einem Geräteteil dort. Mit diesem Restimpuls kann sich das Projektil dann bereits in Hinrichtung bewegen, wird aber bei Einsetzen der ersten Ventilöffnungszeit zusätzlich beschleunigt. Der Bewegungsteil (in Hinrichtung) ohne pneumatische Beschleunigung durch das erste Ventil kann also auch am Anfang der Bewegungsstrecke in Hinrichtung liegen (und natürlich kann dabei die erste Ventilöffnungszeit vor der Kollision mit dem Applikator enden).

Ein weiteres Beispiel betrifft eine solche Abstandszeit oder einen Anteil einer solchen Abstandszeit am Ende der Rückbewegung und vor dem Erreichen des distalen Endes der Bewegungsstrecke. Auch dabei kann eine Variation der Abstandszeit einen (mittelbaren) Einfluss auf die Geschwindigkeit des Projektils bei der nachfolgenden Kollision mit dem Applikator haben, nämlich weil schon die eben erwähnte "Reflexion" an dem distalen Ende abhängig von der Projektilgeschwindigkeit beim Erreichen dieses Endes zu einem unterschiedlichen Restimpuls am Beginn der Hinbewegung führen kann.

Ein weiteres Beispiel betrifft eine Variation beider Abstandszeiten, z. B. komplementär zueinander. Im einfachsten Fall kann bei konstanten Längen der Ventilöffnungszeiten und gegebener Wiederholfrequenz eine der beiden Abstandszeiten auf Kosten der anderen verlängert werden.

Zur Vermeidung von Missverständnissen ist klarzustellen, dass der hier verwendete Begriff der Ventilöffnungszeit grundsätzlich sowohl die zeitliche Dauer, als auch die Lage relativ zu zeitlichen Bezugspunkten, insbesondere relativ zu der jeweils anderen Ventilöffnungszeit, beinhaltet. Der Begriff beinhaltet also den Beginn und das Ende der Ventilöffnungszeit sowie den Abstand dazwischen, wenn nicht im Folgenden ausdrücklich nur von der Dauer oder nur von einem Anfangs- oder Endzeitpunkt die Rede ist.

Weiter oben wurde die Kombination zweier Schaltventile angesprochen, die eine Möglichkeit für eine erfindungsgemäß vorgesehene Doppelventileinrichtung darstellt. Bei dieser Variante sind die beiden Ventile (vorzugsweise unabhängig voneinander) von der Steuereinrichtung ansteuerbar. Alternativ kann aber auch ein einheitliches Ventil verwendet werden, das hier als "Kombinationsventil" bezeichnet wird und das abhängig von der Ansteuerung durch die Steuereinrichtung mindestens zwei Schaltzustände aufweist, nämlich einen ersten zur Beaufschlagung des Projektils in Richtung zu dem Applikator hin und einen zweiten zur Beaufschlagung in der Rückrichtung. Während das Kombinationsventil in dem ersten Schaltzustand ist, ist also eine erste Ventilöffnungszeit gegeben, und im zweiten Schaltzustand dementsprechend eine zweite Ventilöffnungszeit.

In diesen beiden Schaltzuständen wird der jeweils im anderen Schaltzustand zu beaufschlagende pneumatische Anschluss durch das Kombinationsventil vorzugsweise belüftet, sodass also z. B. bei der Hinbewegung auf der zu dem Applikator proximalen Seite des Projektils ungefähr Umgebungsdruck herrscht und im Unterschied zu dem konventionellen Vorgehen mit einer Gegendruckkammer kein von Kollision zu Kollision zunehmender Staudruck.

Auch bei dem Einsatz zweier separater Ventile ist vorzugsweise mindestens eines der beiden Ventile ein "Zweiwegeventil", das dementsprechend eine Belüftung durchführt, sofern es nicht zur Beaufschlagung mit dem pneumatischen Druck geschaltet ist. Weitere Schaltzustände sind allerdings nicht ausgeschlossen und das Ventil ist nicht zwingend auf genau zwei Schaltzustände begrenzt.

Mit einer Belüftung ist im Übrigen eine pneumatisch gut leitfähige Verbindung zur Außenatmosphäre oder einem dieser im Wesentlichen entsprechenden Referenzdruckvolumen gemeint. Es geht also nicht um eine bewusste Verzögerung des Abfließens von Gas und Überdruck im Sinn einer Drosselung.

Alternativ zu einer Belüftung über das Kombinationsventil oder die gerade angesprochenen Zweiwegeventile könnte das Gerät auch z. B. eine gewisse pneumatische Undichtigkeit haben und in Abwesenheit einer Beaufschlagung mit pneumatischem Druck so oder anders quasi kriechend selbst eine gedrosselte Entlüftung durchführen. Diese Möglichkeit ist aber weniger bevorzugt.

Vorzugsweise ist die erste Einschaltzeit bei mindestens zwei Steuerungszuständen mit unterschiedlicher Abstandszeit variabel lang, also z. B. bei einem festen Anteil der Abstandszeit nach der Kollision mit dem Applikator (einschließlich Null). Im einfachsten Fall gibt es dabei nur zwei Steuerungszustände, vorzugsweise aber mehr, wobei natürlich bei einem Steuerungszustand auch eine Abstandszeit Null existieren kann, soweit es zumindest einen anderen mit von Null verschiedener Abstandszeit gibt.

Natürlich kann die zweite Einschaltzeit ebenfalls variabel lang sein, aber z. B. bei dem gerade beschriebenen Fall eines festen Anteils der Abstandszeit nach der Kollision mit dem Applikator durchaus auch konstant sein, und zwar vorzugsweise hinsichtlich Dauer und /oder Anfang und Ende in Bezug auf die Referenz.

Eingangs war bereits davon die Rede, dass Geräte des hier betrachteten Typs typischerweise eine Vielzahl von Beschleunigungs-, Kollisions- und Rücktransportvorgängen des Projektils durchführen. Im Stand der Technik ist die entsprechende Wiederholfrequenz eines solchen periodischen Betriebs regelmäßig einstellbar. Auch vorliegend werden ein iterativer (nicht zwingend periodischer) Betrieb und eine entsprechende Auslegung des Geräts bevorzugt betrachtet, wobei die entsprechende Folge nicht zwingend mit einer Bewegung in der Hinrichtung beginnen muss.

Z. B. kann eine solche Folge mit einem ersten pneumatischen Druckpuls zur Bewegung des Projektils in der Rückrichtung in seine gegenüber dem Applikator distale Position beginnen, um definierte Anfangsbedingungen herzustellen. Grundsätzlich sind zwar Magnete an diesem distalen Ende der Bewegungsstrecke des Projektils im Stand der Technik vorbekannt, mit denen das Projektil fixiert werden soll. Jedoch könnte sich ein Projektil aus dieser Fixierung in Folge von Stößen lösen und es könnte natürlich auch auf eine solche Fixierung verzichtet werden.

Im Übrigen beziehen sich die Erläuterungen nicht zwingend auf jeden einzelnen Bewegungsvorgang während einer solchen Bewegungsfolge. Die Betriebsbedingungen können, wie weiter unten noch näher erläutert, während einer Folge auch verändert werden, sodass die beschriebene Abstandszeit bei einem Teil der Bewegungsabläufe in der Folge möglicherweise gar nicht existiert.

Weiter oben wurden Einzelheiten der vorliegenden Erfindung im Hinblick auf eine Abstandszeit zwischen der ersten und der zweiten Einschaltzeit erläutert. Gemäß einer weiteren bevorzugten Ausgestaltung können das Gerät und insbesondere die Steuereinrichtung so ausgelegt sein, dass in bestimmten weiteren Steuerungszuständen keine solche Abstandszeit sondern stattdessen zwischen der ersten und der zweiten Einschaltzeit oder umgekehrt eine Überlappzeit besteht (die von Null und damit dem Fall einer Abstandszeit Null verschieden ist). Mit einer solchen Überlappzeit in bestimmten Steuerungszuständen (bei existierenden anderen Steuerungszuständen mit Abstandszeit) ergibt sich ein zusätzlicher Freiheitsgrad. Z. B. kann dieser dazu genutzt werden, durch Veränderung der Überlappzeit die Aufschlaggeschwindigkeit des Projektils beim Aufschlagen auf den Applikator zu steuern. Wenn nämlich bei diesem Beispiel zu einem Zeitpunkt vor der Kollision noch während der ersten Ventilöffnungszeit bereits die zweite Ventilöffnungszeit beginnt, so wirkt auf das Projektil neben der in Hinrichtung beschleunigenden pneumatischen Kraft eine Gegenkraft. Diese kann im einfachsten Fall bei Einsatz ungefähr des gleichen pneumatischen Drucks ungefähr gleich groß sein und die Beschleunigung quasi neutralisieren. Je nach der Größe des Anteils der ersten Ventilöffnungszeit vor diesem Zeitpunkt wird das Projektil auf eine größere oder kleinere Geschwindigkeit beschleunigt, die es dann während der Überlappzeit z. B. ungefähr beibehält.

Im umgekehrten Fall gilt Ähnliches: Wenn die Überlappzeit dadurch entsteht, dass z. B. die zweite Ventilöffnungszeit nicht nur bis maximal zur vollständigen Rückkehr des Projektils in die Ausgangsposition genutzt wird, sondern auch noch darüber hinaus etwas andauert, aber die erste Einschaltzeit während der zweiten Einschaltzeit schon beginnt, so wirkt während dieser Überlappzeit auf das Projektil (im einfachsten Fall) wieder keine wesentliche Kraft. So kann es also (eventuell schon während der Rückbewegung und) nach Abschluss der Rückbewegung eine Phase ohne pneumatische Vorwärtsbeschleunigung des Projektils geben, weil diese erst nach Ende der Überlappzeit einsetzt.

Ein Vorteil kann darin bestehen, dass die Aufschlaggeschwindigkeit des Projektils dabei genauer und/oder leichter gesteuert werden kann als im konventionellen Vergleichsfall, in dem es auf die Öffnungszeit des (einzigen) Schaltventils und natürlich den effektiven Druck ankam. Reale Schaltventile haben nämlich endlich lange Öffnungs- und Schließzeiten, öffnen und schließen also nicht instantan. Das gilt vor allem für den Vergleich zwischen der Schließzeit und der Öffnungszeit z. B. bei (hier bevorzugten) federbeaufschlagten Ventilen, bei denen der Öffnungsvorgang magnetisch und optional unter Verwendung des zu schaltenden Drucks pneumatisch unterstützt wird, wohingegen der Schließvorgang durch eine beim Öffnen gespannte Feder erfolgt. Erfahrungsgemäß kann es bauartbedinge und auch alterungsbedingte Abweichungen sowie ein unterschiedliches Alterungsverhalten zwischen den Öffnungs- und Schließzeiten (hier Öffnungszeit im Sinne des Öffnungsvorgangs) geben.

Bei dem ersten obigen Fall einer Überlappzeit am Ende einer ersten Ventilöffnungszeit und am Beginn einer zweiten Ventilöffnungszeit ist der für die Projektilbeschleunigung relevante Zeitraum, nämlich der Anteil der ersten Ventilöffnungszeit vor dieser Überlappzeit, durch die Zeitdifferenz zwischen zwei Ventilöffnungsvorgängen bedingt (zuerst des ersten und danach des zweiten Ventils). Im zweiten obigen Fall liegt die Überlappzeit am Ende der zweiten Ventilöffnungszeit. Hier wird die pneumatische Projektilbeschleunigung in Richtung Applikator erst mit dem Ende der Überlappzeit wesentlich, sodass es um den Restanteil dieser ersten Ventilöffnungszeit nach der Überlappzeit und damit um die Differenz zwischen zwei Ventilschließvorgängen (zuerst des zweiten und danach des ersten Ventils) geht. In beiden Fällen handelt es sich also um die Zeitdifferenz zwischen gleichartigen Ventilbewegungen.

Die Erfinder haben festgestellt, dass sich in dieser Weise vor allem alterungsbedingte Abweichungen deutlich weniger bemerkbar machen (wenn nämlich z. B. die Schließzeit stärkere Alterungseinflüsse zeigt als die Öffnungszeit), weil sich diese Einflüsse durch die beschriebene Differenzbildung zumindest teilweise kompensieren.

In dem zitierten Dokument aus dem Stand der Technik ist hingegen (bei aller Knappheit der hier einschlägigen Darstellung) von einer alternierenden Betriebsweise der beiden erwähnten Ventile auszugehen, was auch zu den in diesem Dokument angestrebten besonders hohen Intensitäten der Projektil-Applikator-Kollisionen und damit Druckwellen passt.

Ein weiterer (alternativer oder zusätzlicher) Aspekt kann darin liegen, einerseits mit einem relativ großen pneumatischen Druck zu arbeiten, um eine schnelle Rückführung und damit auch eine hohe Betriebsfrequenz zu erzielen, andererseits dabei aber nicht zwingend diesem hohen Druck entsprechende hohe Aufschlaggeschwindigkeiten (bei Anstehen eines solchen Beschleunigungsdrucks während der gesamten Hinbewegung) verwenden zu müssen. Hohe Intensitäten sind aus therapeutischen Gründen nicht immer gewünscht und im Übrigen regelmäßig mit einer erhöhten Belastung des Patienten durch Schmerzen oder andere Reizungen verbunden.

Bei dem ersten oben beschriebenen Fall, nämlich einer Überlappzeit am Ende einer ersten Einschaltzeit, werden die zweite Einschaltzeit und damit die Überlappzeit während einer Hinbewegung des Projektils begonnen, gibt es also eine Schlussphase dieser Hinbewegung, in der beidseits des Projektils Druck ansteht. Daraus ergeben sich die bereits erläuterten Möglichkeiten. Allerdings ist dieses Merkmal nicht obligatorisch, denn auch (nur) nach dem Aufschlagen, also während oder zu Beginn der Rückbewegung des Projektils, kann ein beidseitiges Anstehen von Druck sinnvoll sein. Beispielsweise kann der wegen der Beschleunigung des Projektils gewünschte Versorgungsdruck für die Rückbewegung etwas üppig bemessen sein, etwa weil es unerwünscht ist, das Projektil distal vom Applikator mit ähnlicher Wucht aufschlagen zu lassen wie auf dem Applikator. In diesem Sinn kann z. B. eine Gleichzeitigkeit von erster und zweiter Ventilöffnungszeit zu Beginn der Rückbewegung eine gewisse Drosselwirkung hinsichtlich der Rückbewegung haben.

Bei dem zweiten oben beschriebenen Fall, nämlich einer Überlappzeit am Ende einer zweiten Einschaltzeit kann mit einem Teil der Überlappzeit noch während der Rückbewegung ein ähnlicher Effekt erreicht werden wie gerade beschrieben. Vergleichbares gilt auch für eine solche Gleichzeitigkeit gegen Ende der Rückbewegung, also vor dem nächsten Beschleunigungsvorgang. Insbesondere kann die Überlappzeit teils vor und anderenteils nach dem Aufschlagen des Projektils auf dem Applikator oder auch teils vor und anderenteils nach dem Erreichen des von dem Applikator fernsten Punkts liegen, wie dies im Zusammenhang mit dem Ausführungsbeispiel noch näher erläutert wird.

Ferner ist es in dem ersten Fall der Überlappzeit am Ende der ersten Einschaltzeit bevorzugt, die erste Einschaltzeit während der zweiten Einschaltzeit zu beenden und somit die zweite Einschaltzeit typischerweise über die erste Einschaltzeit hinaus andauern zu lassen. Insbesondere gilt das natürlich für die beschriebene Rückführung des Projektils. Allerdings ist auch dieses Merkmal nicht zwingend. Z. B. kann die beschriebene Kompensation der beschleunigenden Kraft durch eine Gleichzeitigkeit von erster und zweiter Einschaltzeit (also beidseitige Beaufschlagung des Projektils mit pneumatischem Druck) auch durch eine vergleichsweise kurze zweite Einschaltzeit vollständig innerhalb einer ersten Einschaltzeit erfolgen. Dann kann z. B. in einer aus dem Stand der Technik bereits bekannten Weise das Projektil rückgeführt werden. Grundsätzlich könnte für die Rückführung sogar eine weitere zweite Einschaltzeit (die von der zweiten Einschaltzeit während der ersten getrennt ist) benutzt werden.

Analog ist es in dem zweiten Fall einer Überlappzeit am Ende der zweiten Einschaltzeit ebenfalls bevorzugt, die zweite Einschaltzeit während der (nächsten) ersten Einschaltzeit enden zu lassen, insbesondere um damit den bereits beschriebenen pneumatischen Beschleunigungsvorgang des Projektils in Richtung Applikator zu bewirken.

Vorzugsweise kann eine der beiden Einschaltzeiten beim Vergleich zwischen zwei Steuerungszuständen mit unterschiedlicher Überlappzeit (in diesem Fall einschließlich Null) konstant lang sein. In dem Fall einer Überlappzeit am Ende der ersten Einschaltzeit betrifft dies vorzugsweise diese erste Einschaltzeit und im anderen Fall entsprechend die zweite Einschaltzeit. Das bedeutet also, dass sich in diesen Fällen die Unterschiede der Überlappzeit aus unterschiedlichen Längen der jeweils damit überlappenden anderen Ventilöffnungszeit oder den zeitlichen Verhältnisse zwischen den beiden Ventilöffnungszeiten ergeben. Vorzugsweise ist die jeweils andere Ventilöffnungszeit zwischen zwei solchen Steuerungszuständen mit unterschiedlicher Überlappzeit in der Dauer variabel (wobei sie z. B. bei allen oder einem Teil der Steuerungszustände gemessen von dem Beginn der früheren Einschaltzeit aus den gleichen Anfangszeitpunkt haben könnte).

Vorzugsweise ist der Anteil der Überlappzeit vor dem Aufschlagen des Projektils, also bei der Hinbewegung des Projektils von einem (relativ zu dem Applikator) distalen Ort zu dem Applikator, deutlich größer als der auf die Rückbewegung entfallende Anteil bzw. Zeitraum. Dies gilt vorzugsweise für alle Steuerungszustände mit einer Überlappzeit. Wenn der Aspekt einer gewissen Hemmung der Heftigkeit einer Rückbewegung nicht von Bedeutung ist, kann es außerdem bevorzugt sein, dass die Überlappzeit ausschließlich während der Hinbewegung existiert.

Die pneumatische Einrichtung kann im einfachsten Fall einen Anschluss zur Versorgung aus einem pneumatischen Leitungsnetz, z. B. in einem Krankenhaus, oder aus einer Druckgasflasche aufweisen. Bevorzugt ist jedoch ein Pneumatikkompressor, mit dem das erfindungsgemäße Gerät örtlich unabhängig und im Vergleich zu einer Druckgasflasche mobiler ist. An sich sind Pneumatikkompressoren im Zusammenhang mit solchen Geräten vorbekannt. Die Erfindung bietet jedoch den besonderen Aspekt, bei verschiedenen Steuerungszuständen mit unterschiedlichen Aufschlaggeschwindigkeiten des Projektils nicht zwingend den Versorgungsdruck ändern zu müssen. In anderen Worten kann der Kompressor in solchen verschiedenen Steuerungszuständen bei gleicher Drehzahl laufen.

Natürlich kann dadurch zunächst einmal die Ansteuerung des Kompressors vereinfacht werden, insbesondere wenn dieser grundsätzlich im eingeschalteten Zustand immer bei der gleichen Drehzahl läuft. Ferner kann der Kompressor dabei in der Nähe seines oder bei seinem Effizienzmaximum (hinsichtlich der Drehzahl) betrieben werden. Überdies ist es möglich, Geräuschminderungsmaßnahmen, z. B. eine dämpfende Lagerung des Kompressors oder eine geräuschdämmende Umhüllung, auf das Schwingungsverhalten des Kompressors bei der einen gleichen Drehzahl abzustimmen.

Eine besondere Gestaltungsmöglichkeit der Erfindung liegt darin begründet, allein durch Veränderung von Ventilöffnungszeitpunkten oder Ventilöffnungszeitdauern die Stoßphysik zwischen Projektil und Applikator direkt und schnell beeinflussen zu können, und zwar die Aufschlaggeschwindigkeit und damit den Impuls beim Aufschlag. Im Vergleich zu einer Veränderung des Versorgungsdrucks ist diese Einflussnahmemöglichkeit besonders schnell, sodass in einem iterativen Betriebszustand prinzipiell von einem Aufschlagprozess zum nächsten die/der Aufschlaggeschwindigkeit/-impuls der kombinierten Hin- und Rückbewegung verändert werden kann. Eine so schnelle und freie Einflussnahme erlaubt der Stand der Technik nicht.

Typische Aufschlaggeschwindigkeiten liegen dabei, aber auch bei sich weniger schnell oder nicht verändernden Bedingungen, im Bereich von zwischen 2 m/s und 30 m/s. Für die Stoßphysik ist vor allem der Aufschlagimpuls wichtig, der bei typischen Projektilmassen zwischen 1 g und 10 g, vorzugsweise zwischen 2 g und 5 g, somit in einem Bereich von 2 gm/s bis 300 gm/s liegen kann. Bevorzugt ist ein Bereich zwischen 10 gm/s und 150 gm/s liegen kann.

Bei einer besonderen Ausgestaltung weist das Gerät eine Messeinrichtung auf, mit der der Durchtritt des Projektils an einer Stelle seiner Bewegungsstrecke gemessen werden kann. Diese Messeinrichtung kann mit der Steuereinrichtung gekoppelt sein. Damit kann in solcher Form z. B. der Durchtritt des Projektils kurz vor dem Aufschlagen oder quasi beim Aufschlagen auf den Applikator erfasst werden, sodass die Einschaltzeiten dementsprechend (insbesondere hinsichtlich ihres Anfangs und ihres Endes) auf den Endzeitpunkt des Aufschlagens abgestimmt werden können.

Eine solche Erfassung kann z. B. optisch erfolgen, etwa durch eine Lichtschranke oder dergleichen, vorzugsweise aber induktiv unter Verwendung mindestens einer Messspule. Diese kann durch einen Restmagnetismus des Projektils oder rein induktiv (durch Veränderung der Streuinduktivität) das Projektil erfassen.

Im Folgenden wird die Erfindung anhand von Ausführungsbeispielen näher erläutert, wobei die einzelnen Merkmale im Rahmen der Ansprüche auch in anderer Kombination erfindungswesentlich sein können.

Im Einzelnen zeigt
- Figur 1: eine perspektivische Darstellung eines erfindungsgemäßen Geräts, wobei ein mittlerer Gehäuseteil der Übersichtlichkeit halber weggelassen ist;
- Figur 2: einen Längsschnitt durch das Gerät aus Figur 1 in gegenüber Figur 1 rechts-links-vertauschter Lage;
- Figur 3: ein schematisches Diagramm des Handstücks mit einem zugehörigen Basisgerät;
- Figur 4: eine Folge schematischer Zeitverlaufsdiagramme 4a) bis f) zur Erläuterung der Funktionsweise;
- Figur 5: eine schematische Darstellung eines Kombinationsventils zur Erläuterung eines alternativen Ausführungsbeispiels zu den Figuren 1 und 2;
- Figur 6: eine Folge schematischer Zeitverlauf Diagramme 6a) bis e) zur Erläuterung weiterer Steuerungszustände in Ergänzung zu Figur 4;
- Figur 7: ein weiteres schematisches Verlaufsdiagramm zur Erläuterung der periodischen Arbeitsweise;
- Figur 8: eine wiederkehrende Folge von zwei verschiedenen Projektilgeschwindigkeitsniveaus in direkter Abfolge, wobei nach einem Puls mit hoher Geschwindigkeit zwei Pulse mit niedriger Geschwindigkeit direkt nachfolgen;
- Figur 9: eine Steuersequenz für die Ansteuerung der Ventile V1 und V2 gemäß der in Figur 8 gezeigten Folge von zwei verschiedenen Projektilgeschwindigkeitsniveaus;
- Figur 10: eine höhere zeitliche Detaillierung der ersten 300 ms aus der Steuersequenz von Figur 9.

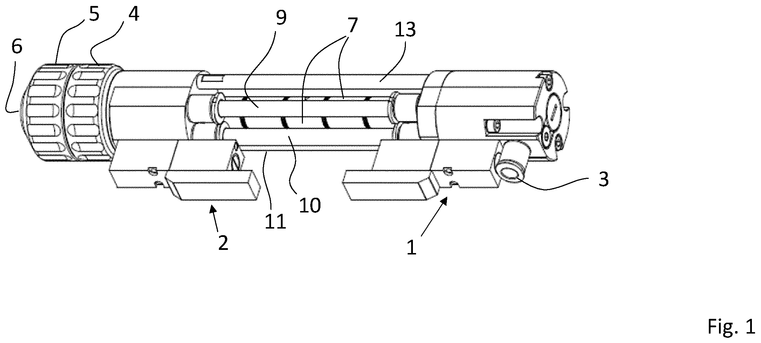

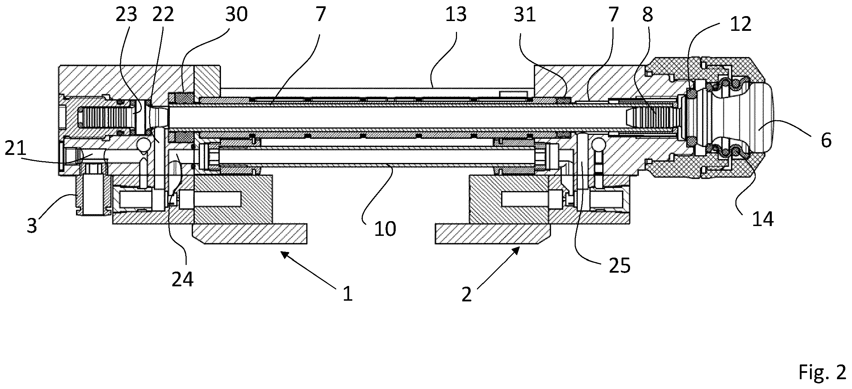

Figur 1 zeigt ein Handstück eines erfindungsgemäßen Geräts in perspektivischer Ansicht mit nach vorn-links weisenden pneumatischen Ventilen, nämlich einem ersten Ventil 1 und einem zweiten Ventil 2. Rechts sieht man einen pneumatischen Versorgungsanschluss 3 und links zwei zur leichteren Handhabung jeweils außen geriffelte Schraubringe 4 und 5 zur Halterung des im Folgenden noch näher erläuterten Applikators 6. Dieser ist in Figur 1 ganz links mit seiner patientenzugewandten Oberfläche gerade noch zu sehen und im Übrigen in Figur 2 gezeigt. Er könnte auch mehrteilig aufgebaut sein.

Im mittleren Bereich des Geräts aus Figur 1 erkennt man eine Mehrzahl in Querrichtung laufender Röhren, wobei die mittlere mit dem Bezugszeichen 7 das in Figur 2 im Schnitt erkennbare Projektil 8 enthält und führt. Davor sieht man zwei parallele pneumatische Verbindungsrohrleitungen 9 und 10 zwischen den beiden Ventilen 1 und 2, wobei die Rohrleitung 9 zum Zuführen einer Druckbeaufschlagung zu dem zweiten Ventil 2 und die Rohrleitung 10 umgekehrt zur Entlüftung dieses zweiten Ventils 2 über einen bei dem ersten Ventil 1 vorgesehenen Auslass dient. Diese Mehrzahl Rohre ist bei diesem Ausführungsbeispiel von einer in Figur 1 durch die Linie unter der Rohrleitung 10 und die beiden Linien über dem Projektilführungsrohr 7 dargestellten Gehäuseabdeckung 11 umgeben. Diese Gehäuseabdeckung 11 läuft in Figur 1 im hinteren Bereich und umfasst nur einen Teil des Umfangs. Sie ist an ihren jeweiligen axialen Kanten ähnlich einer Bördelung durch ein verrundetes Umschlagen nach innen griffgünstig gestaltet, was in Figur 1 bei der oberen Kante angedeutet ist. Die Gehäuseabdeckung 11 kann also bei der praktischen Handhabung als Griff dienen. Der Abstandshalter 13 stabilisiert den Aufbau und verbindet die beiden Enden des Handstücks mechanisch.

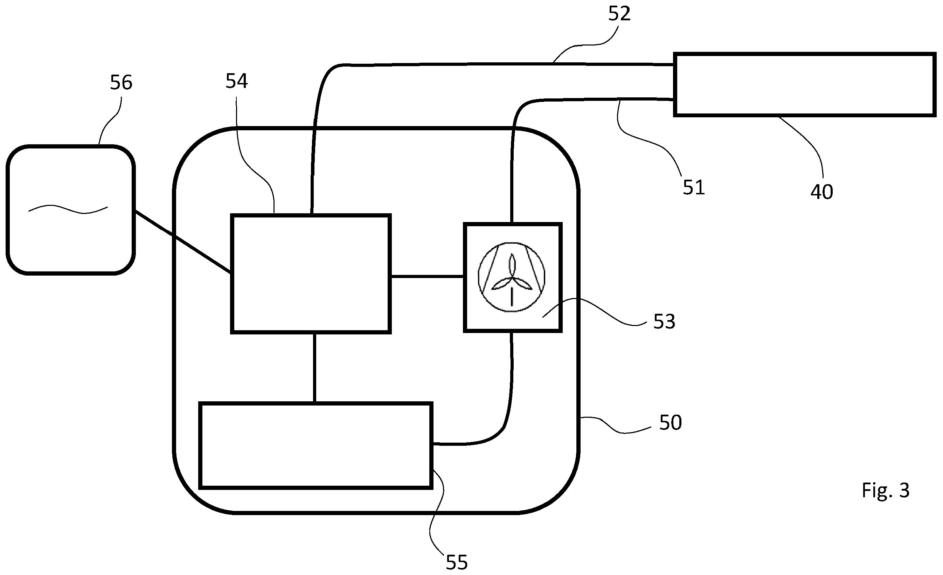

Eine von einem Pneumatikkompressor zu dem Gerät führende flexible Druckluftzuleitung (vgl. 51 in Figur 3) ist hier nicht eingezeichnet und an den bereits erwähnten Anschluss 3 anzuschließen. Analog ist eine elektronische Steuerleitung (52 in Figur 3) von einer externen Steuerung zu den Ventilen 1 und 2 nicht eingezeichnet, die mit der Druckluftzuleitung einheitlich ausgeführt sein kann.

Figur 2 zeigt einen Längsschnitt entlang einer gedachten Mittellängsachse der bereits erwähnten Zylinderform des Gesamtgeräts, die gleichzeitig eine Mittellängsachse des Projektilführungsrohrs 7 ist. In diesem Projektilführungsrohr ist das Projektil 8 in Figur 2 rechts eingezeichnet und damit in Anlage an dem Applikator 6, der von dem beschriebenen Schraubring 4 und 5 in einer an sich bekannten Weise gehalten ist. Dabei ist der Applikator 6 mit einem balgartigen Elastomerring 14 in Axialrichtung elastisch gelagert und mit einem weiteren Elastomerring 12 pneumatisch abgedichtet. Alternativ ist auch ein Geräteaufbau hinsichtlich des Applikators 6 und seiner Halterung und Abdichtung gemäß z. B. der EP 2 529 679 (auch unabhängig von der dort gezeigten Kappe) oder der EP 2 095 843 (auch unabhängig von dem dort thematisierten Material Keramik) möglich und bevorzugt.

Figur 2 zeigt links einen inneren Kanal 21, der den Pneumatikanschluss 3 mit dem ersten Ventil 1 verbindet. Das erste Ventil 1 kann demzufolge einen an dem Pneumatikanschluss 3 anstehenden Versorgungsdruck steuerungsabhängig auf einen radialen Kanal 22 schalten, der unter einem Dämpferelement 23 mündet und damit an das Innenvolumen des Projektilführungsrohrs 7 angeschlossen ist. Über diesen Kanal 22 wird also das Projektil während einer ersten Einschaltzeit in Richtung zu dem Applikator 6 hin beaufschlagt bzw. beschleunigt. Unabhängig davon wird der pneumatische Versorgungsdruck über den Kanal 24 und das Rohr 10 an das zweite Ventil 2 weitergegeben.

In der zweiten alternativen Schaltstellung werden der Kanal 22 und damit auch das Innenvolumen des Projektilführungsrohrs 7 zwischen dem distalen Ende (in Figur 2 links) und dem Projektil 8 belüftet.

In dem zweiten Ventil 2, das grundsätzlich spiegelsymmetrisch zu dem ersten Ventil 1 aufgebaut ist, kann der über das Rohr 10 anliegende pneumatische Versorgungsdruck alternativ über den Kanal 25 radial nach oben gegeben werden zu einem das Projektilführungsrohr 7 umgebenden Volumen (in Figur 2 als Schlitz über und unter dem Rohr 7 zu erkennen), das von dem Anschluss des Kanals 25 aus nach rechts, also in Richtung zu dem Applikator 6, führt und dort zwischen dem Applikator 6 und dem zu ihm proximalen Ende des Projektilführungsrohrs 7 an das Innenvolumen des Projektilführungsrohrs 7 angeschlossen ist (von der in Figur 2 gezeichneten Anwesenheit des Projektils 8 dort abgesehen). Über den Kanal 25 kann also der pneumatische Versorgungsdruck schaltbar an das Innenvolumen des Projektilführungsrohrs 7 zwischen dem Applikator 6 und dem Projektil 8 angelegt werden. Dabei ist allerdings bei diesem Beispiel die pneumatische Verbindung in Folge eines kleineren effektiven Öffnungsquerschnitts etwas schlechter, als an der entgegengesetzten Seite des Projektilführungsrohrs 7, so dass sich hier bei größeren Luftströmungsgeschwindigkeiten (höhere Frequenzen, größere Drücke) früher oder stärker Verzögerungen bemerkbar machen.

Alternativ kann das zweite Ventil 2 in der anderen Schaltstellung den Anschluss des Innenvolumens des Rohrs 10 an ihm sperren und den Kanal 25 und damit das Innenvolumen des Projektilführungsrohrs 7 rechts vom Projektil 8 belüften, also über eine pneumatisch gut leitfähige Verbindung an die Außenatmosphäre anschließen.

Die beiden Ventile 1 und 2 können mithin das Projektil von beiden Seiten mit pneumatischem Druck beaufschlagen, und zwar unabhängig voneinander und damit gleichzeitig oder wechselseitig oder können das Innere des Projektilführungsrohrs 7 beiderseits belüften.

Das Bezugszeichen 30 in Figur 2 bezeichnet einen ringförmigen Permanentmagneten am gegenüber dem Applikator 6 distalen Ende der (mit der Länge des Projektilführungsrohrs 7 zusammenfallenden) Bewegungsstrecke des Projektils 8. Mit diesem Magneten 30 kann das aus ferromagnetischem Material aufgebaute Projektil 8 an diesem distalen Ende der Bewegungsstrecke leicht fixiert werden. Durch einseitige Druckbeaufschlagung mittels des Ventils 2 kann das Projektil ferner in diese Position zurückgeführt und optional auch zusätzlich dort gehalten werden, insbesondere bei Betriebsbeginn oder bei nicht ferromagnetischem Projektil. Insoweit kann der Permanentmagnet 30 optional auch weggelassen werden, vor allem dann, wenn die im weiteren Verlauf noch erläuterten Reflexionen an diesem distalen Ende der Bewegungsstrecke auch bei kleinen Aufprallgeschwindigkeiten des Projektils 8 dort ermöglicht werden sollen.

Mit 31 ist eine Stelle beziffert, an der man mit einer Messspule den Durchtritt des Projektils 8 durch die entsprechende Stelle der Bewegungsstrecke erfassen könnte, wobei diese Stelle relativ nah an dem Applikator 6 liegt. Im einfachsten Fall nutzt man hier einen leichten Restmagnetismus des Projektils 8 aus, aber man könnte natürlich auch die Veränderung der Induktivität der Spule 31 wechselstromtechnisch erfassen und auswerten. Man kann die Kollision des Projektils 8 mit dem Applikator 6 auch durch die Verwendung eines Mikrophons oder Bewegungssensors im Versuchsaufbau ermitteln. Außerdem kann man im Versuchsaufbau z. B. mit zwei kurz vor dem Applikator 6 positionierten Lichtschranken die Aufprallgeschwindigkeit des Projektils 8 ermitteln.

Figur 3 zeigt ein Blockschema mit dem in den Figuren 1 und 2 dargestellten Gerät rechts oben, und zwar mit dem Bezugszeichen 40 summarisch bezeichnet. Dies Gerät 40 ist ein in der Hand zu haltendes mobiles Handstück, wie es von einschlägigen Geräten aus dem Stand der Technik an sich bereits bekannt ist. Es ist über zwei Leitungen 51 und 52 an eine Basisstation 50 angeschlossen, welche einen Pneumatikkompressor 53 und eine Steuerung 54 enthält. Der Kompressor 53 ist über die Leitung 51, nämlich eine pneumatische flexible Schlauchleitung, mit dem Handgerät 40 verbunden und die Steuerung 54 über die (optional mit der Leitung 51 integrierte) elektrische Leitung 52, über die die Steuerung auf die bereits erwähnten beiden Ventile 1 und 2 zugreifen und diese mit Strom versorgen kann. Über die Leitung 52 kann außerdem eine Kommunikation mit dem Handstück 40 erfolgen, insbesondere wenn dort zusätzlich eine Steuerung bzw. ein Teil der Steuerung vorgesehen ist.

Die Steuerung 54 steuert im Übrigen auch den Kompressor 53 hinsichtlich seiner Drehzahl und natürlich des Ein- und Ausschaltens und wird ihrerseits, genauso wie der Kompressor 53, von einem Netzgerät 55 leistungsversorgt. In dem Kompressor 53 kann aber auch eine die Drehzahl beeinflussende Druckregelung oder ein Regelventil integriert sein. Außerdem ist die Steuerung 54 an ein Display 56 angeschlossen, das in dem Basisgerät 50 eingebaut oder auch separat davon implementiert sein kann. Das Basisgerät 50 wird über einen berührungsempfindlichen Bildschirm 56 und/oder über eine hier nicht dargestellte Anordnung von Tasten bedient.

Der Benutzer kann also die Funktion des Geräts 40 anhand solcher Tasten und jedenfalls anhand des Displays 56 steuern, wobei die Steuerung 54 insbesondere die Öffnungs- und Schließzeiten und damit auch die Öffnungsdauern der beiden Ventile 1 und 2 vorgibt. Teilaufgaben der Steuerung 54 können auch im Handstück 40 integriert sein, besonders was die Ansteuerung der Ventile 1 und 2 betrifft.

Zum Grundverständnis der Ansteuerung der beiden Ventile kann verwiesen werden auf das ältere Patent EP 2 213 273 B1. Das Ausführungsbeispiel darin entspricht hinsichtlich der Dimensionierung insbesondere des Projektilführungsrohrs und des Projektils weitgehend den obigen Erläuterungen und den Figuren 1 und 2 mit Ausnahme der Existenz des zweiten Ventils 2 und des Wegfalls der Gegendruckkammer. Außerdem wird in dem zitierten Ausführungsbeispiel von einer bestimmten Ventilöffnungszeit des dort einzigen Ventils bei einem bestimmten Druck ausgegangen, wohingegen die Projektilbeschleunigung im vorliegenden Fall durch den Anteil der ersten Ventilöffnungszeit auch außerhalb der Überlappzeit und damit auch bei einem konstanten Druck variabel erfolgt. Für die folgenden Erläuterungen kann beispielhaft von einem Druck von 2 bar und einer Wiederholfrequenz von 10 Hz ausgegangen werden. Damit ergibt sich folgende Wertetabelle mit gemessenen Werten:

| | | | | | | |
|---|---|---|---|---|---|---|
| Projektilgeschwindigkeit [m/s] | 4.7 | 6.2 | 8.1 | 9.5 | 10.3 | 10.8 |
| Öffnungszeitpunkt Ventil 1 [ms] | 0 | 0 | 0 | 0 | 0 | 0 |
| Schließzeitpunkt Ventil 1 [ms] | 13 | 13 | 13 | 13 | 13 | 13 |
| Öffnungszeitpunkt Ventil 2 [ms] | 13 | 14 | 15 | 16 | 17 | 18 |
| Schließzeitpunkt Ventil 2 [ms] | 21 | 22 | 23 | 24 | 25 | 26 |
| Aufschlagzeitpunkt [ms] | 25.0 | 24.6 | 24.2 | 23.9 | 23.8 | 23.7 |

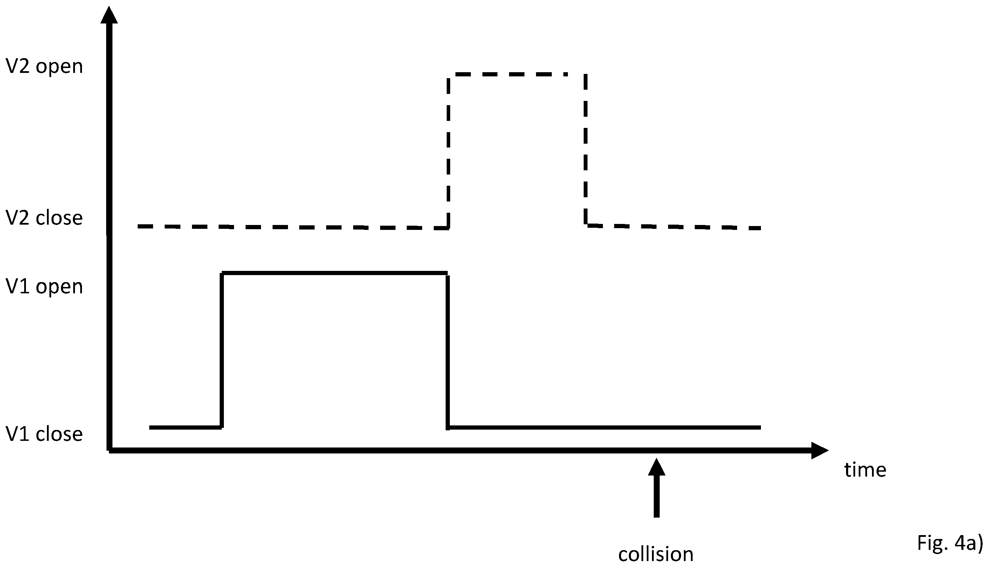

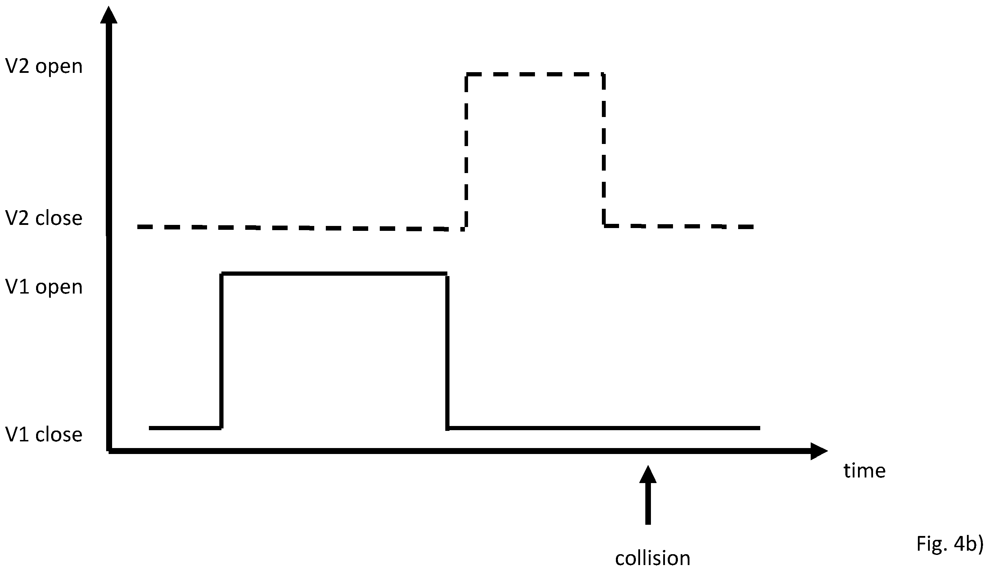

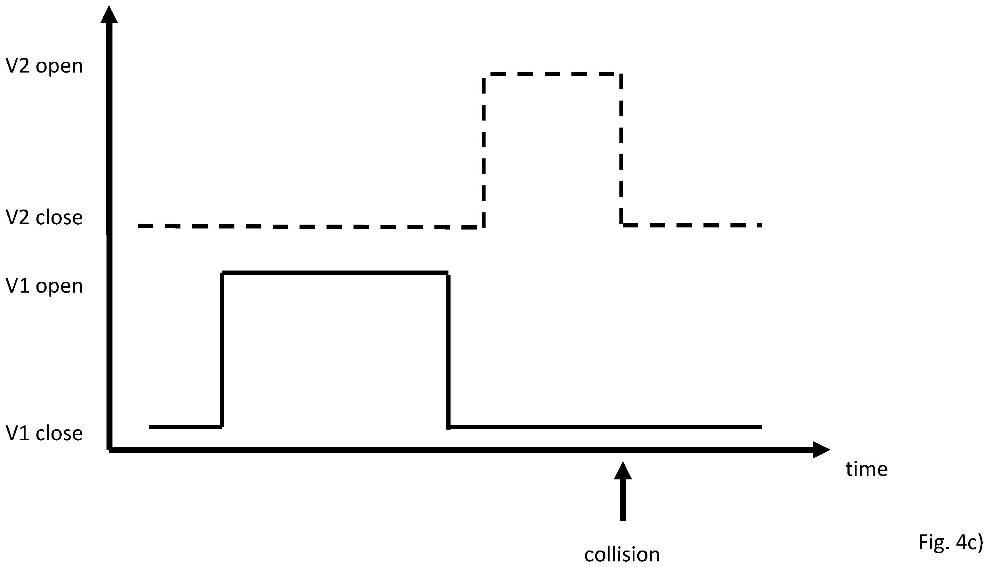

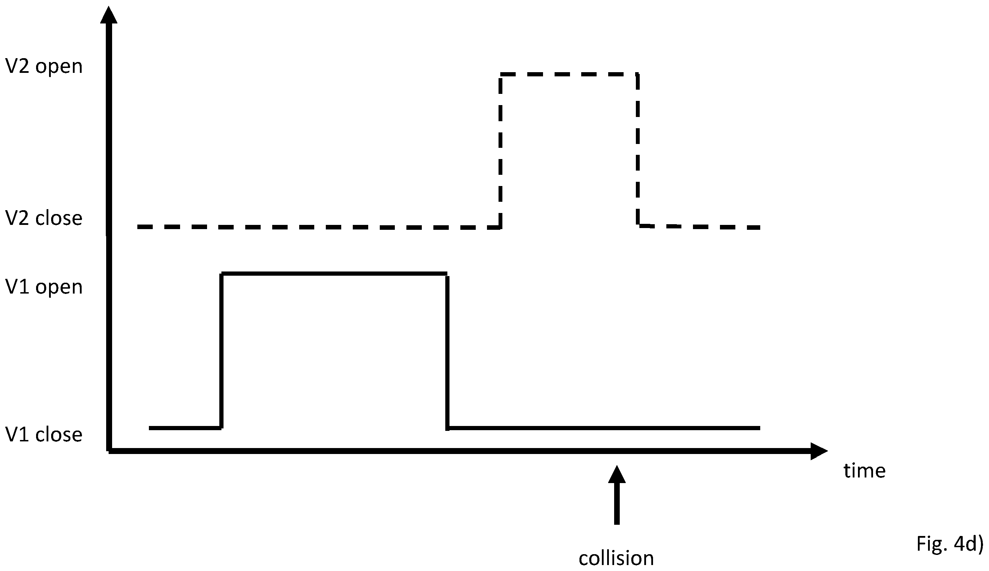

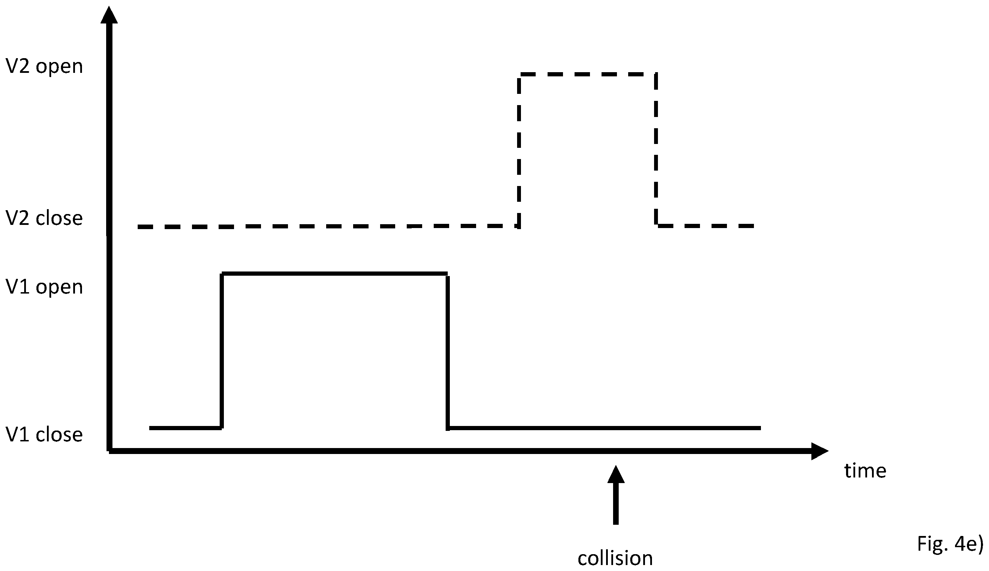

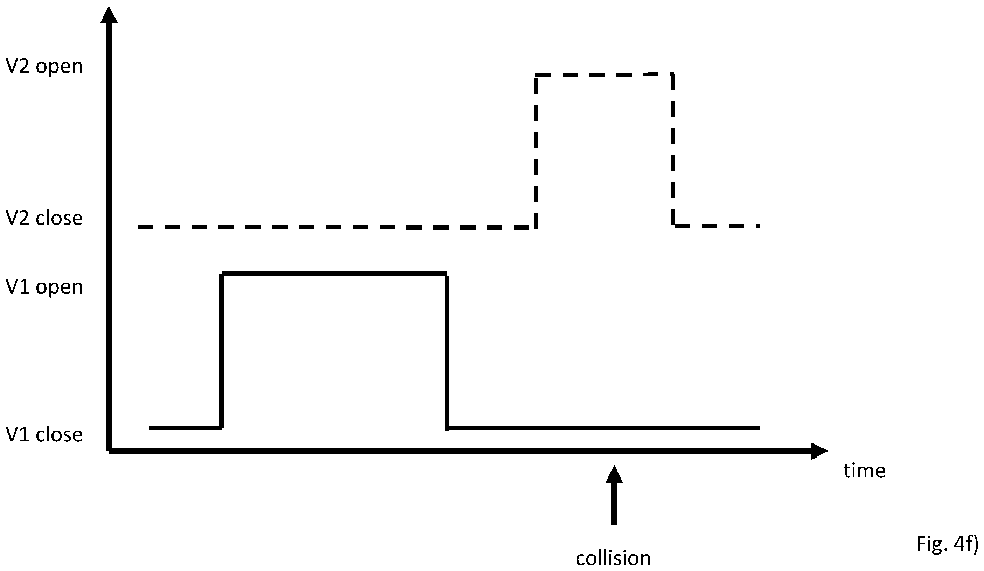

Figur 4 zeigt in den Einzeldarstellungen a) bis f) schematische Zeitverlaufsdiagramme, die der obigen Tabelle entsprechen. Es gibt verschiedene Abstandszeiten zwischen der in durchgezogener Linie unten repräsentierten Ansteuerung des Ventils 1 aus den Figuren 1 und 2 und der in gestrichelter Linie oben dargestellten Ansteuerung des Ventils 2. Es gibt generell einen Einschaltpuls für das Ventil 1, womit das Projektil 8 beschleunigt wird und dann nach dem Ende dieser ersten Einschaltzeit für einen unterschiedlichen Teil der Bewegungsstrecke ohne weitere pneumatische Beaufschlagung "weiterfliegt". In dieser Bewegungsphase sind beide Seiten des Rohrinneren belüftet (und nicht druckbeaufschlagt). In Figur 4a) gilt das nur nach der zweiten Einschaltzeit.

Nach einer bestimmten Zeit der Abbremsung durch das zweite Ventil kommt es zu einer eingezeichneten Kollision mit dem Applikator 6 und danach zu der Rückbewegung des Projektils 8 aufgrund dieser Kollision und des rückführenden pneumatischen Pulses infolge (des Rests) der zweiten Einschaltzeit. Damit wird das Projektil 8 wieder in die Ausgangslage zurückbewegt.

Die Länge der ersten Einschaltzeit wird unverändert gelassen. Bei diesem Beispiel liegt mindestens ein Teil der zweiten Einschaltzeit vor der Kollision, und zwar in den Fällen a) bis e) die ganze zweite Einschaltzeit oder der überwiegende Teil, im Fall f) ungefähr die Hälfte.

Diese Darstellungen veranschaulichen eine weitere Möglichkeit der Steuerung der Geschwindigkeit des Projektils 8 bei der Kollision. In Figur 4a) wird nämlich das Projektil 8 über die erste Einschaltzeit hinweg pneumatisch beschleunigt, um sodann von einem entgegenstehenden pneumatischen Druck infolge des Beginns der zweiten Einschaltzeit (gestrichelt oben) verzögert zu werden. Da im Fall a) die Verzögerungszeit im Verhältnis 8:13 zur Beschleunigungszeit steht und von der gleichen Druckhöhe ausgegangen werden kann, schlägt das Projektil 8 mit einer minimalen Geschwindigkeit auf dem Applikator 6 auf und wird nach dem Aufprall auf dem Applikator 6 aufgrund des elastischen Stoßes wieder zum Ausgangspunkt zurückbewegt.

In den Fällen b) bis f) ist die Abstandszeit zwischen den beiden Einschaltzeiten größer und damit der Anteil der zweiten Einschaltzeit vor der Kollision schrittweise kleiner, was trotz unveränderter erster Einschaltzeit zu einer zunehmenden Projektilgeschwindigkeit bei der Kollision führt.

Genau genommen zeigen die Figuren 4a) bis f) die elektrischen Steuerzeiten der beiden Ventile 1 und 2, also die Ausgangssignale der Steuerung 54. Die Ventile 1 und 2 sind federunterstützte Magnetventile, die rein magnetisch öffnen und durch die Kraft der dabei gespannten Feder schließen, wenn der Magnet nicht mehr beaufschlagt wird. Die Bewegungen des Ventilkörpers sind dementsprechend gegenüber den dargestellten Steuersignalen etwas verzögert, und zwar um schätzungsweise 4 ms beim Öffnen und 2 ms beim Schließen.

Bei einem sogenannten Pilotventil mit pneumatischer Unterstützung beim Öffnen wäre die Situation qualitativ vergleichbar.

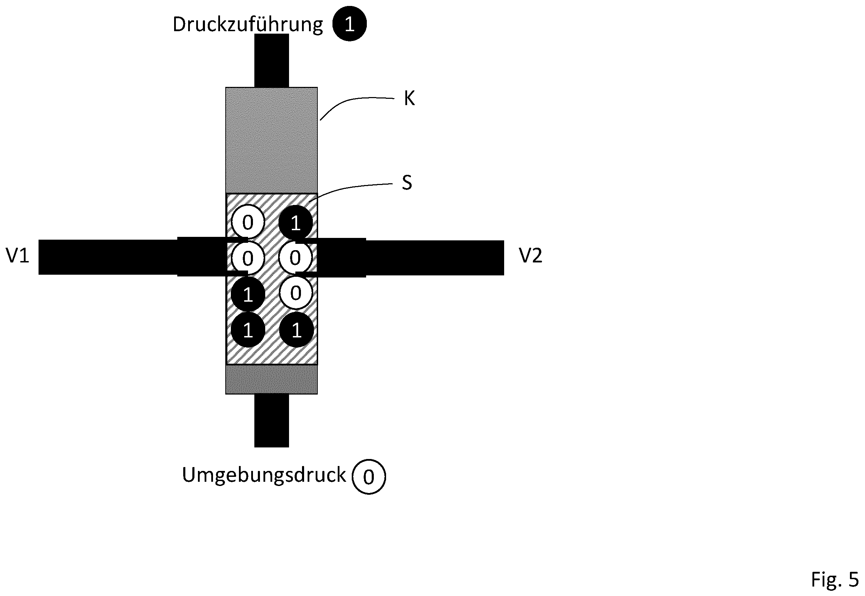

Natürlich kann man bei einem anderen Ausführungsbeispiel mit einem "Kombinationsventil" ganz ähnliche Verhältnisse erzeugen wie in Figur 4 in den Diagrammen a) bis f) dargestellt. Ein solches Kombinationsventil ist in Figur 5 schematisch dargestellt. Dabei bezeichnet der Buchstabe K das Kombinationsventil, das dementsprechend die beiden Ventile 1 und 2 aus den Figuren 1 und 2 ersetzt. Rechts und links sind zwei Leitungen V1 und V2 dargestellt, von denen V1 einen Anschluss an die linke Seite (gemäß Figur 2) des Projektilführungsrohrs 7, z. B. über das Kanalstück 22 (analog zu dem ersten Ventil 1) bedeutet. Dementsprechend bedeutet die rechte Leitung V2 einen Anschluss an die rechte Seite des Projektilführungsrohrs 7 (analog zu dem zweiten Ventil 2), also z. B. über das Kanalstück 25.

Die obere Leitung ist in Figur 5 mit dem Stichwort "Druckzuführung" und dem Symbol "1" (nicht zu verwechseln mit dem Bezugszeichen 1) für das erste Ventil bezeichnet; analog ist der untere Leitungsanschluss mit dem Stichwort "Umgebungsdruck" und dem figureninternen Symbol "0" bezeichnet, bedeutet also eine Belüftungsöffnung.

In dem Kombinationsventil K gibt es einen symbolisch dargestellten Schieber S, der in der vertikalen Richtung (bezogen auf Figur 5) zwischen vier verschiedenen Schaltpositionen verschoben werden kann. In der obersten ist, wie die Figur 5 verdeutlicht, der Anschluss V1 belüftet und der Anschluss V2 mit dem pneumatischen Versorgungsdruck beaufschlagt, in der dritten von oben umgekehrt und in der gerade aktiv geschalteten zweiten Position von oben sind beide Anschlüsse V1 und V2 belüftet. Schließlich zeigt die unterste Position eine gleichzeitige Druckbeaufschlagung beider Anschlüsse V1 und V2, vgl. weiter unten.

Man könnte sich also ein in dieser oder ähnlicher Weise aufgebautes Kombinationsventil K anstelle der beiden einzelnen Ventile 1 und 2 aus dem Ausführungsbeispiel in den Figuren 1 und 2 vorstellen, wobei die übrigen Erläuterungen und insbesondere die Figuren 3 und 4 sinngemäß auch dafür gelten.

Wegen der Steuerungsmöglichkeit der Aufschlaggeschwindigkeit des Projektils 8 allein über den Schaltbetrieb der beiden Ventile 1 und 2 läuft der Pneumatikkompressor 53 (Figur 3) bei einer vorgegebenen festen Betriebsfrequenz, in der er ein Wirkungsgradmaximum hat. Außerdem kann der Pneumatikkompressor bei einer vorgegebenen Betriebsfrequenz besonders wirksam schwingungs- und geräuschgedämmt werden.

Grundsätzlich kann die Steuereinrichtung 54 die Aufschlaggeschwindigkeit und auch den Zeitabstand zwischen den Kollisionen zwischen dem Projektil 8 und dem Applikator 6 von einem zu nächsten Einzelvorgang verändern. Sie kann also deutlich schneller und variabler die Stoßphysik beeinflussen und ist insbesondere nicht an periodische Vorgänge gebunden.

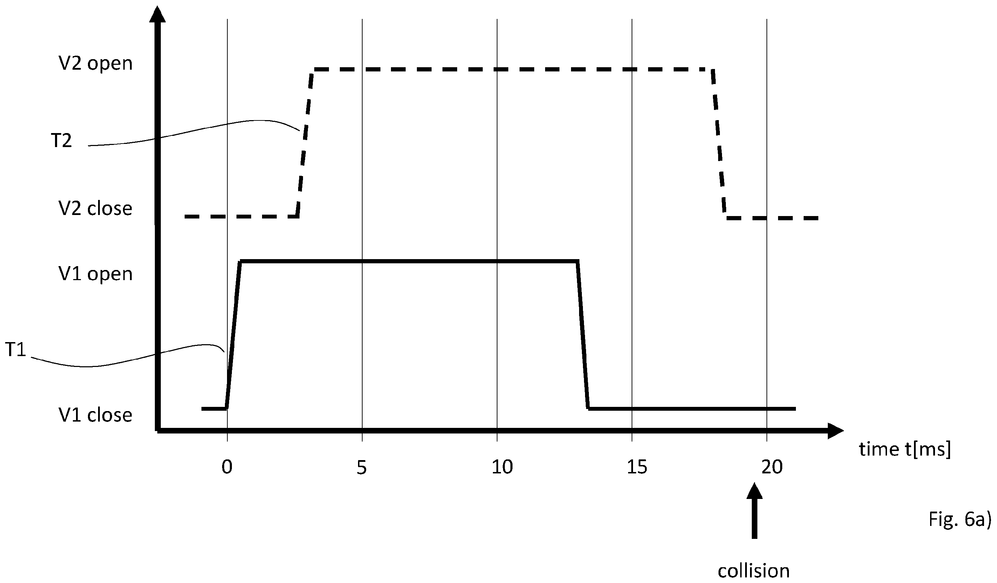

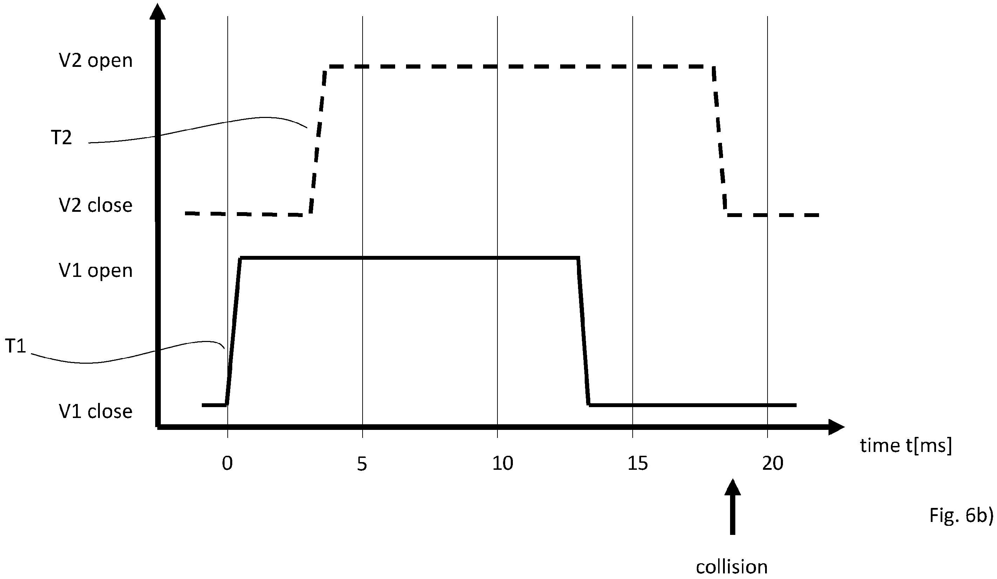

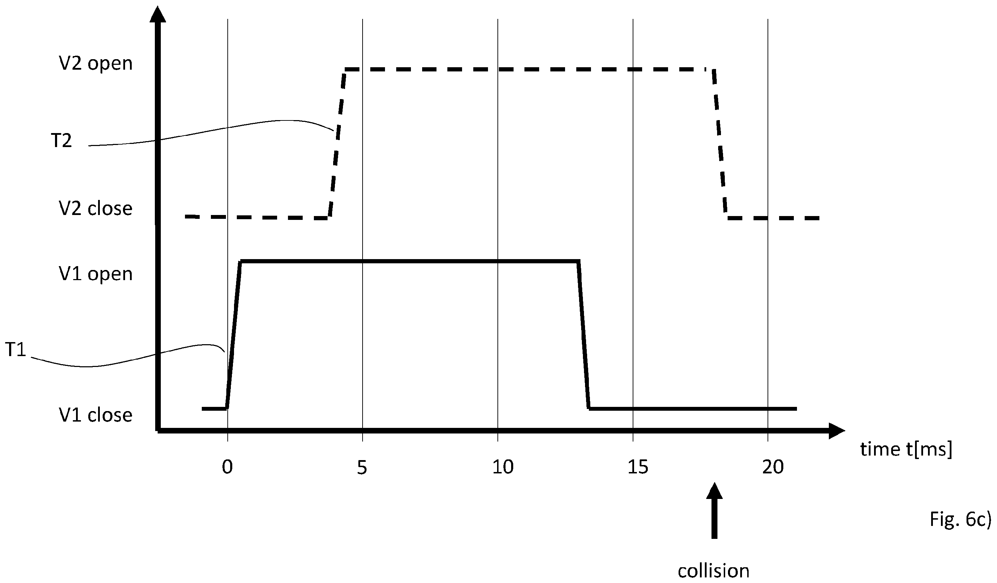

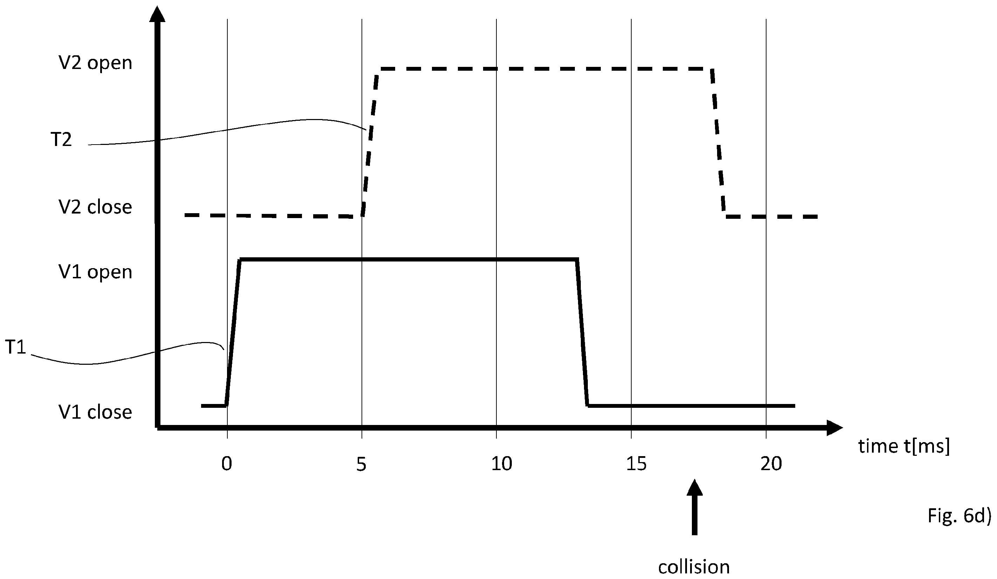

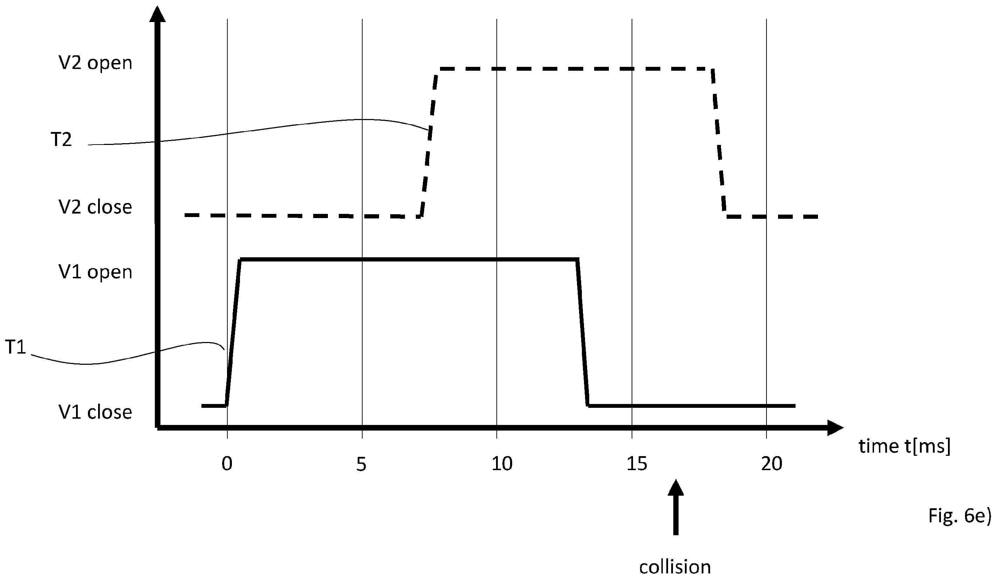

Figur 6 zeigt eine Folge von fünf einzelnen schematischen Zeitverlaufsdiagrammen 6a) bis 6e), in denen jeweils mit der mit T1 bezeichneten Kurve der Öffnungs- und Schließvorgang des ersten Ventils 1 und mit der mit T2 bezeichneten Kurve analog der Öffnungs- und Schließvorgang des zweiten Ventils T2 bezeichnet ist. Der erhöhte Kurventeil entspricht also jeweils der ersten/zweiten Einschaltzeit.

Im Vergleich erkennt man, dass die erste Einschaltzeit bei allen fünf Steuerungszuständen auf der (willkürlichen) Zeitachse in der horizontalen bei 0 ms beginnt und bei 13 ms endet. Demgegenüber verschiebt sich die zweite Einschaltzeit hinsichtlich ihres Beginns von anfangs etwa 2,5 ms in Figur 6a) schrittweise bis etwa 7 ms in Figur 6e), wohingegen die zweite Einschaltzeit in allen fünf Darstellungen bei etwa 18 ms endet. Dementsprechend gibt es in allen Steuerungszuständen eine Überlappzeit, nämlich von 3 ms bis 13 ms in Figur 6a) bis hin zu noch von 7 ms bis 13 ms in Figur 6e), wobei diese Überlappzeit schrittweise abnimmt, nämlich entsprechend dem zunehmend verzögerten Beginn der zweiten Einschaltzeit. Insoweit ist in allen fünf Steuerungszuständen die pneumatische Beaufschlagung durch das zweite Ventil 2 hinsichtlich der Rückführung des Projektils 8 aktiv.

In den in Figur 6 dargestellten Fällen werden bei einem Druck von 4 bar Aufschlaggeschwindigkeiten des Projektils 8 auf dem Applikator 6 von (in dieser Reihenfolge von a) zu e)) 10 m/s, 12 m/s, 14 m/s, 16 m/s und 18 m/s realisiert. Das entspricht Impulsen von 30 gm/s bis 54 gm/s bei einer Projektilmasse von 3 g. Die Einschaltzeit des Ventils 1 beträgt konstant 13,0 ms. Konstant bleibt auch der Schließzeitpunkt des zweiten Ventils bei 18 ms Die Einschaltzeit des zweiten Ventils verändert sich (wieder in der Folge von a) zu e)) von 15,4 ms über 15,0 ms, 14,3 ms, 13,0 ms zu 10,9 ms, woraus sich Überlappzeiten von 10,4 ms, 10,0 ms, 9,3 ms, 8,0 ms und schließlich 5,9 ms ergeben. Dementsprechend beginnt die Einschaltzeit des zweiten Ventils 2 um einen (von oben nach unten zunehmenden) Zeitraum zwischen 2,6 ms und 7,1 ms gegenüber der ersten Einschaltzeit verzögert.

In Figur 6a) (natürlich bei einem Start der Projektilbewegung am linken Ende der Bewegungsstrecke in Figur 2 bei 0 ms) erfolgt die Kollision mit dem Applikator nach der Überlappzeit und auch nach dem Ende der zweiten Einschaltzeit, also bei ungefähr 18 ms bis 20 ms, wobei sich dieser Kollisionszeitpunkt in den folgenden Figuren immer weiter nach links verlagert und ab Figur 6c) innerhalb der zweiten Einschaltzeit liegt. Die (in einem Versuchsaufbau optisch) gemessenen Projektilgeschwindigkeiten betragen zwischen 10 m/s in Figur 6a) und 18 m/s in Figur 6e) und stehen damit in einem Verhältnis von 1:1,8.

Man kann sich dabei vereinfacht vorstellen, dass das Projektil vor der zweiten Einschaltzeit linear über die Zeit beschleunigt wird und danach mit etwa der erreichten Geschwindigkeit weiter bewegt wird (unter Vernachlässigung von pneumatischen Strömungseffekten und Projektilreibung); tatsächlich wird die Projektilgeschwindigkeit mit der Zeit wohl etwas weniger als linear zunehmen und in einem angenähert kräftefreien Zustand während der Überlappzeit reibungsbedingt leicht abnehmen. Nach der Überlappzeit wird das Projektil 8 in allen Einzeldarstellungen durch die noch anstehende pneumatische Beaufschlagung durch das zweite Ventil gebremst, wobei das Projektil in den Fällen 6a) und 6b) nach dem Ende der zweiten Einschaltzeit wieder im obigen Sinne annähernd kräftefrei ein kurzes Stück bis zur Kollision zurücklegt.

Vor allem in Figur 6a) fällt auf, dass der Rest der zweiten Einschaltzeit nach der Überlappzeit deutlich länger ist der (anfängliche) Rest der ersten Einschaltzeit vor der Überlappzeit. Dies mag verwundern, weil an beiden Ventilen der gleiche Versorgungsdruck anliegt und ausweislich der realen Werte aus der obigen Wertetabelle trotzdem eine Kollision in Figur 6a) mit immerhin 10 m/s stattfindet. Ein Grund dürfte darin liegen, dass das zweite Ventil 2 gemäß Figur 2 im Bereich des rechten Endes des Projektilführungsrohres 7 pneumatisch deutlich ineffizienter an das Innere dieses Rohres 7 angeschlossen ist als das erste Ventil 1 am linken Ende. Das hat damit zu tun, dass am rechten Ende, wie in Figur 2 zu erkennen, das Projektil 8 durch eine Querschnittsverengung (Fangeinrichtung) vor einem Herausfliegen nach rechts bewahrt wird. Das hat Sicherheitsgründe, wenn versehentlich ein Gerät ohne montierten Applikator in Betrieb gesetzt werden würde. Insoweit füllt sich das entsprechende Rohrende beim Öffnen des zweiten Ventils 2 offenbar langsamer und ist damit bei dynamischer Betrachtung eine größere Verzögerung zwischen den Ventilschaltvorgängen und der tatsächlichen Kraftausübung aufgrund der pneumatischen Beaufschlagung durch das zweite Ventil gegeben.

Im Übrigen zeigen die Figuren, dass nur in den Darstellungen 6d) und 6e) ein Anteil der zweiten Einschaltzeit nach der Kollision liegt. Das stört nicht weiter, weil das Projektil durch die Kollision selbst im Sinn des Stoßes zwischen einem typischerweise masseärmeren Projektil und einem massestärkeren Applikator im Sinn der Impulserhaltung zurückgestoßen wird. Der Rest der zweiten Einschaltzeit nach Ende der ersten Einschaltzeit in Figuren 6d) und e) stellt insoweit die Rückbewegung in die Ausgangslage nur zusätzlich sicher.

Natürlich könnte man die Steuerzeiten insoweit anpassen, dass die Überlappzeit ungefähr jeweils zum Kollisionszeitpunkt endet. Insbesondere könnte das mit einer zeitlichen Bestimmung des Kollisionszeitpunkts durch die anhand Figur 2 bereits veranschaulichte Möglichkeit einer Messspule 31 in der Nähe des Applikators 6 geschehen. Das Steuerzeitenschema würde damit etwas komplizierter, weil die erste Einschaltzeit unterschiedlich früh beendet werden müsste (von Figur 6a) zu Figur 6e) immer früher). Es könnte allerdings die Geschwindigkeit der Projektilrückbewegung erhöht werden und damit jedenfalls für die späteren der Einzeldarstellungen, also für die höheren Projektilgeschwindigkeiten, auch ein noch höherer Wiederholfrequenzbereich erreichbar werden, indem nämlich auch das Ende der zweiten Einschaltzeit dementsprechend unterschiedlich und bei zunehmender Projektilgeschwindigkeit früher vorgesehen wird.

Im Übrigen sind auch hier elektrische Steuerzeiten dargestellt, sodass aus den geschilderten Gründen tatsächlich etwa 2 ms kürzere Überlappzeiten bestehen.

Insgesamt muss man sich eine Steuerung (gemäß Figur 3) vorstellen, die Steuerungszustände gemäß den Teildarstellungen in Figur 4 und weitere Steuerungszustände gemäß den gerade erläuterten Teildarstellungen in Figur 6 einstellen kann. In beiden Fällen kann die Projektilgeschwindigkeit bei der Kollision durch Ventilschaltzeitpunkte bei gleichbleibendem Druck beeinflusst werden.

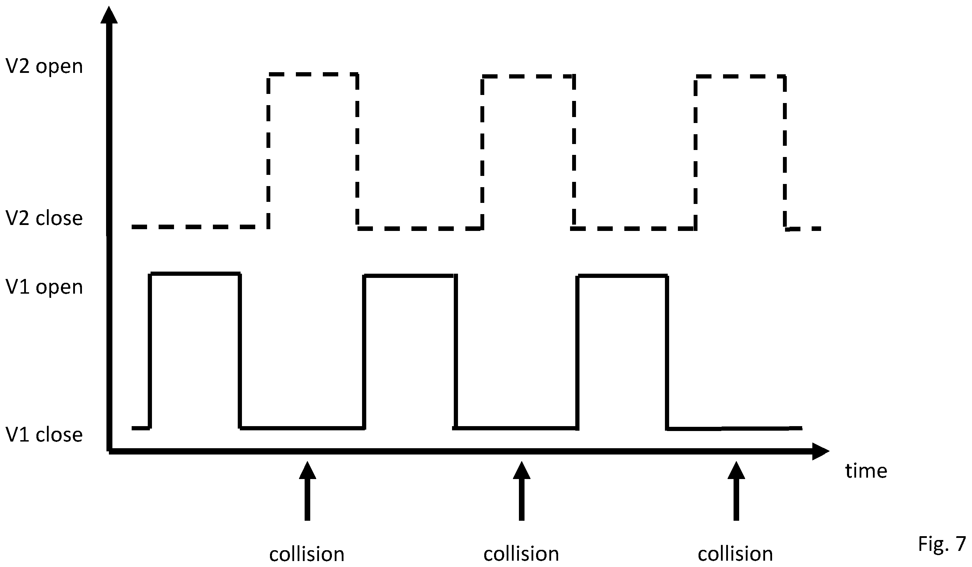

Figur 7 zeigt ungefähr eine Folge von drei Vorgängen entsprechend Figur 4f). Dabei wird durch die gestrichelt eingezeichneten zweiten Einschaltzeiten das Projektil 8 jeweils wieder in die Ausgangslage zurückgebracht, um dann von der zeitlich folgenden ersten Einschaltzeit wieder Richtung Applikator 6 beschleunigt zu werden. Diese Figur soll lediglich die mögliche Periodizität von Steuerungszuständen veranschaulichen, was in analoger Weise natürlich auch für die anderen Teildarstellungen in den Figuren 4 und 6 gilt. Außerdem kann man sich vorstellen, dass die aufeinanderfolgenden Prozesse Abweichungen voneinander haben können, sodass also der Stoßprozesses von einem zum nächsten Wiederholungsvorgang schnell und frei verändert werden kann.

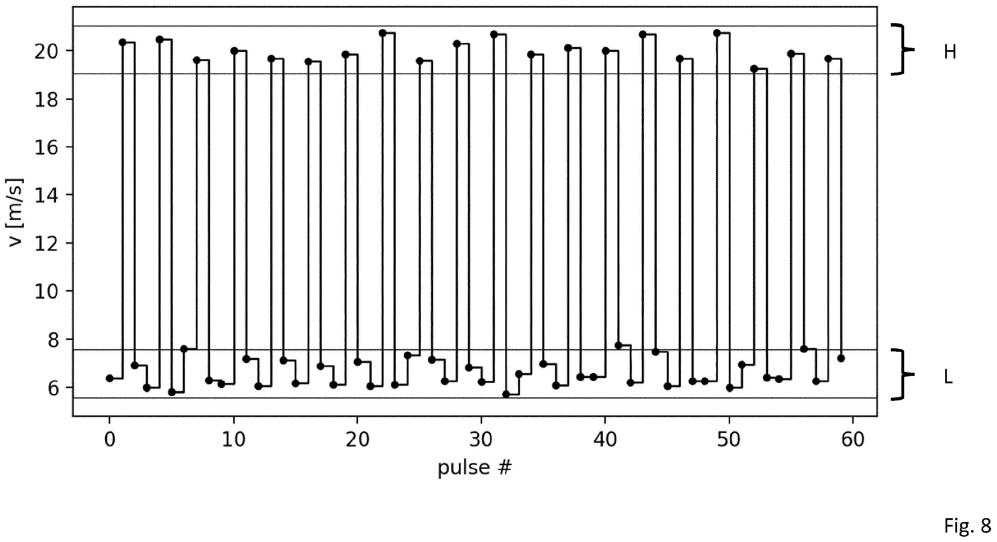

Figur 8 zeigt eine wiederkehrende Folge von Pulsen mit zwei unterschiedlichen Projektilgeschwindigkeitsbereichen (beim Aufprall), die in der Figur 8 durch die Bezugszeichen H und L gekennzeichnet sind. Durch Variation von Überlapp und Abstand der Öffnungszeiten kann hier exemplarisch die Leistungsfähigkeit der Steuerung gezeigt werden. Auf jeweils einen Puls mit einer Projektilgeschwindigkeit etwa im Bereich H, kommen zwei Pulse mit einer Projektilgeschwindigkeit etwa im Bereich L.

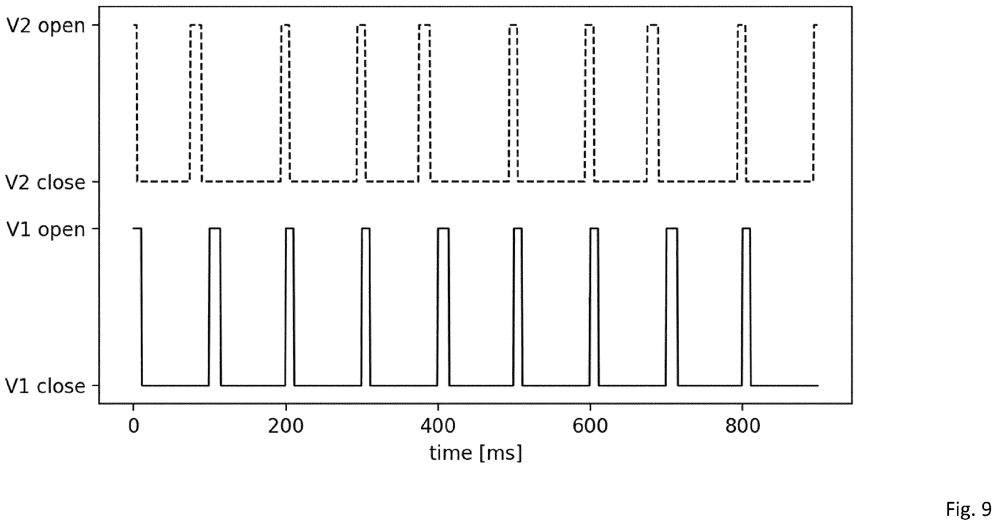

Figur 9 demonstriert insbesondere, dass die Kollisionsbedingungen von einer Kollision zur nächsten wesentlich geändert werden können, hier mit ungefähr einem Faktor 3 in der Kollisionsgeschwindigkeit. Die Schwankungen innerhalb der Bereiche H und L sind dabei unbeabsichtigte und toleranzbedingte Streuungen (es handelt sich um reale Messwerte).

Figur 9 zeigt exemplarisch die Steuersequenz für die Ventile V1 und V2 in ihrer zeitlichen Abfolge, um die Projektilgeschwindigkeitsfolgen zu erzielen, welche in Figur 8 zu sehen sind. Es sind unterschiedliche Überlappungen und Abstände von Pulsen relativ zueinander zu sehen.

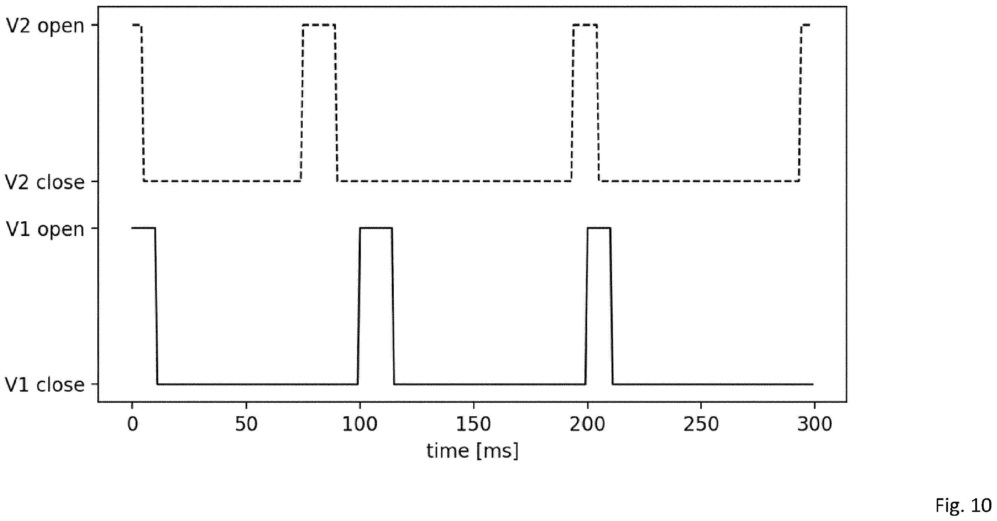

Figur 10 stellt die Abfolge der Pulse aus Figur 9 zeitlich genauer auf, sodass hier eine sich wiederholende Sequenz einzeln zu sehen ist. Hier ist zu sehen, dass Pulse zwischen V1 und V2 relativ ihren Abstand und Überlapp ändern.

Die obigen Erläuterungen beziehen sich auf das in den Figuren 1 bis 3 dargestellte Gerät. Sie lassen sich anhand einfacher Abschätzungen zur Projektilbewegung auch auf andere Geräte und Dimensionen übertragen. Insbesondere sind die Umkehrpunkte der Projektilbewegung bspw. über die erwähnte Messspule, evtl. eine analoge Messspule am distalen Ende der Bewegungsstrecke oder über die Erfassung der Kollisionen per Mikrophon, leicht zugänglich. Auf dieser Grundlage lassen sich anhand der obigen Schilderungen sinnvolle Abschätzungen treffen.

Alternativ kann folgendermaßen vorgegangen werden: Man gibt eine gewünschte Betriebsfrequenz und einen gewünschten Versorgungsdruck für die beiden Ventile vor und gibt z. B. auch vor, dass beide Ventile für konstante Dauer öffnen, z. B. für 25 % des Kehrwerts der vorgegebenen Frequenz. Dann kann man die Steuerung so einrichten, dass die Ventile in einem Startzeitpunkt genau gleichphasig öffnen und schließen. In diesem Zustand wird keine stabile Bewegung zustande kommen, weil das Projektil zeitgleich beidseits mit Druck beaufschlagt wird bzw. von keiner Seite mit Druck beaufschlagt wird. Auf dieser Grundlage kann man dann den Versatz zwischen Öffnungszeitpunkten in beide Richtungen schrittweise verändern, also schrittweise das zweite Ventil etwas früher oder etwas später als das erste Ventil öffnen (und schließen). Ab einem gewissen Zeitversatz, also sozusagen ab einer gewissen Phasenverschiebung, wird es zu einem stabilen Schwingungszustand des Projektils kommen, was man z. B. mit der erwähnten Mikrophonermittlung der Kollisionen an den beiden Enden der Bewegungsstrecke feststellen kann. Außerdem kann man dann die Intensität der Kollision mit dem Applikator ermitteln und die beschriebene Phasenverschiebung gewissermaßen als Stellparameter für die Intensität betrachten. In dieser Form lässt sich eine Eichkurve ermitteln.

Außerdem kann man natürlich bei einem bestimmten in dieser Form ermittelten Schwingungszustand den Phasenversatz konstant halten und die erste und/oder die zweite Ventilöffnungsdauer schrittweise verändern.

Im Einzelfall könnte es dazu kommen, dass für die gewünschte Frequenz kein ausreichender Druck vorgegeben wurde, also auch bei "gegenphasiger" Ansteuerung der beiden Ventile kein Schwingungszustand mit Kollisionen an den Enden der Bewegungsstrecke entsteht. Dann muss dementsprechend entweder etwas der Druck erhöht oder die Frequenz verringert werden.

Analog kann man sich natürlich auch in anderer Form an geeignete Betriebszustände empirisch herantasten. Schließlich lässt sich natürlich das Bewegungsverhalten des Projektils zumindest näherungsweise rechnerisch simulieren und es können dann empirische Versuche ausgehend von den Resultaten solcher Simulationen unternommen werden.

## Patentansprüche

1. Gerät zur Behandlung des menschlichen oder tierischen Körpers mit mechanischen Druckwellen, welches Gerät aufweist:
- ein Projektil (8), das in dem Gerät entlang einer Bewegungsstrecke geführt ist,
- einen Applikator (6) an einem Ende der Bewegungsstrecke,
- eine pneumatische Einrichtung zur Beaufschlagung des Projektils (8) mit pneumatischem Druck zwecks einer Bewegung entlang der Bewegungsstrecke,
wobei das Projektil (8) zu einem Aufschlagen auf den Applikator (6) zur Erzeugung der mechanischen Druckwellen ausgelegt ist,
welche pneumatische Einrichtung eine Doppelventileinrichtung (1,2) zur Beaufschlagung des Projektils (8) mit pneumatischem Druck in Richtung zu dem Applikator (6) hin während einer ersten Einschaltzeit und zur Beaufschlagung des Projektils (8) mit pneumatischem Druck in der Rückrichtung während einer zweiten Einschaltzeit und eine Steuereinrichtung (54) zur Ansteuerung der Doppelventileinrichtung (1,2) aufweist,
**dadurch gekennzeichnet, dass** das Gerät dazu ausgelegt ist, zwischen der ersten Einschaltzeit und der zweiten Einschaltzeit oder umgekehrt eine Abstandszeit einzuhalten und eine Aufschlaggeschwindigkeit des Projektils (8) beim Aufschlagen auf den Applikator (6) mittels der Abstandszeit zu steuern.

2. Gerät nach Anspruch 1, bei dem die Doppelventileinrichtung (1,2) ein erstes Ventil zur Beaufschlagung des Projektils (8) mit pneumatischem Druck in Richtung zu dem Applikator (6) hin und ein zweites Ventil zur Beaufschlagung des Projektils (8) mit pneumatischem Druck in der Rückrichtung aufweist, die vorzugsweise unabhängig voneinander von der Steuereinrichtung (54) ansteuerbar sind.

3. Gerät nach Anspruch 1, bei dem die Doppelventileinrichtung (1,2) ein "Kombinationsventil" aufweist, das abhängig von der Ansteuerung durch die Steuereinrichtung (54) einen ersten Schaltzustand zur Beaufschlagung des Projektils (8) in Richtung zu dem Applikator (6) hin oder einen zweiten Schaltzustand zur Beaufschlagung des Projektils (8) in der Rückrichtung einnimmt, wobei in jedem dieser Schaltzustände der in dem jeweils anderen Schaltzustand zur Beaufschlagung des Projektils (8) verwendete pneumatische Anschluss durch das Kombinationsventil belüftet wird.

4. Gerät nach Anspruch 2, bei dem mindestens eines der beiden Ventile ein Zweiwegeventil ist, das ein pneumatisches Volumen zwischen sich und dem Projektil (8) in einer ersten Schaltstellung während der jeweiligen Einschaltzeit zur Beaufschlagung des Projektils (8) mit pneumatischen Druck beaufschlagt und das in einer zweiten Schaltstellung dieses pneumatische Volumen belüftet.

5. Gerät nach einem der vorstehenden Ansprüche, bei dem die erste Einschaltzeit im Vergleich zwischen mindestens zwei Steuerungszuständen mit unterschiedlicher Abstandszeit variabel lang ist.

6. Gerät nach einem der vorstehenden Ansprüche, dazu ausgelegt, bei einem Teil von Steuerungszuständen die eine der beiden Einschaltzeiten erst nach Beginn der anderen der beiden Einschaltzeiten zu beenden und vorzugsweise die eine Einschaltzeit während der anderen Einschaltzeit zu beenden, so dass die erste und die zweite Einschaltzeit während einer Überlappzeit überlappen.

7. Gerät nach Anspruch 6, dazu ausgelegt, bei dem Teil der Steuerungszustände eine Aufschlaggeschwindigkeit des Projektils (8) beim Aufschlagen auf den Applikator (6) mittels eines Anteils der ersten Einschaltzeit außerhalb der zu ihr gehörenden Überlappzeit zu steuern.

8. Gerät nach Anspruch 7, bei dem die frühere der beiden Einschaltzeiten im Vergleich zwischen mindestens zwei Steuerungszuständen mit unterschiedlichen Zeiträumen des Überlapps zwischen der ersten und der zweiten Einschaltzeit konstant lang ist.

9. Gerät nach Anspruch 7 oder 8, bei dem die spätere der beiden Einschaltzeiten im Vergleich zwischen mindestens zwei Steuerungszuständen mit unterschiedlichen Zeiträumen des Überlapps variabel ist.

10. Gerät nach einem der vorstehenden Ansprüche, bei dem die pneumatische Einrichtung einen Pneumatikkompressor aufweist, wobei das Gerät dazu ausgelegt ist, den Kompressor im eingeschalteten Zustand bei verschiedenen Steuerungszuständen mit unterschiedlichen Aufschlaggeschwindigkeiten des Projektils (8) bei gleicher Drehzahl laufen zu lassen, vorzugsweise grundsätzlich im eingeschalteten Zustand bei immer der gleichen Drehzahl laufen zu lassen.

11. Gerät nach einem der vorstehenden Ansprüche, bei welchem das Projektil (8) mit einem Aufschlagimpuls von zwischen 2 gm/s und 300 gm/s beim Aufschlag auf den Applikator (6) bewegt werden kann.

12. Gerät nach einem der vorstehenden Ansprüche, dazu ausgelegt, in einem iterativen Betriebszustand mit unmittelbar aufeinanderfolgenden Hinbewegung des Projektils (8) zum Aufschlag auf den Applikator (6) und Rückbewegungen die Aufschlaggeschwindigkeit und/oder die Zeitdauer der kombinierten Hin- und Rückbewegung von einer zur nächsten solchen kombinierten Hin- und Rückbewegung zu verändern.

13. Gerät nach einem der vorstehenden Ansprüche mit einer Messeinrichtung zur Erfassung eines Durchtritts des Projektils (8) an einer Stelle der Bewegungsstrecke, welche Messeinrichtung mit der Steuereinrichtung (54) gekoppelt ist.

## Claims

1. Apparatus for treatment of the human or animal body with mechanical pressure waves, the apparatus comprising:
- a projectile (8) guided in the apparatus along a movement path,
- an applicator (6) at one end of the movement path,
- pneumatic means for application of pneumatic pressure to the projectile (8) for the purpose of movement along the movement path,
wherein the projectile (8) is adapted for striking onto the applicator (6) for generating the mechanical pressure waves,
which pneumatic means has a double valve means (1, 2) for application of pneumatic pressure to the projectile (8) in the direction towards the applicator (6) during a first activation time and for application of pneumatic pressure to the projectile (8) in the reverse direction during a second activation time and a control means (54) for controlling the double valve means (1, 2),
**characterized in that** the apparatus is adapted to maintain a separation time between the first activation time and the second activation time or vice versa and to control an impact speed of the projectile (8) upon impact onto the applicator (6) by means of the separation time.

2. Apparatus according to claim 1, in which the double valve means (1, 2) has a first valve for application of pneumatic pressure to the projectile (8) in the direction towards the applicator (6) and a second valve for application of pneumatic pressure to the projectile (8) in the reverse direction, which valves can preferably be controlled independently of one another by the control means (54).

3. Apparatus according to claim 1, in which the double valve means (1, 2) has a "combination valve" which, depending on the control by the control means (54), assumes a first switching state for application of pneumatic pressure to the projectile (8) in the direction towards the applicator (6) or a second switching state for application of pneumatic pressure to the projectile (8) in the reverse direction, wherein in each of these switching states the pneumatic connection used in the respectively other switching state for application of pneumatic pressure to the projectile (8) is ventilated by the combination valve.

4. Apparatus according to claim 2, in which at least one of the two valves is a two-way valve which applies pneumatic pressure to a pneumatic volume between itself and the projectile (8) in a first switching position during the respective activation time for application of pneumatic pressure to the projectile (8) and which ventilates this pneumatic volume in a second switching position.

5. Apparatus according to one of the preceding claims, in which the first activation time is of variable length in comparison between at least two control states with different separation time.

6. Apparatus according to one of the preceding claims, adapted, in the case of a part of control states, to end one of the two activation times only after the start of the other of the two activation times and preferably to end the one activation time during the other activation time, so that the first and the second activation time overlap during an overlap time.

7. Apparatus according to claim 6, adapted, in the case of the part of the control states, to control an impact speed of the projectile (8) upon impact onto the applicator (6) by means of a portion of the first activation time outside the overlap time associated therewith.

8. Apparatus according to claim 7, wherein the earlier one of the two activation times is of constant length in comparison between at least two control states with different periods of overlap between the first and the second activation time.

9. Apparatus according to claim 7 or 8, wherein the later one of the two activation times is of variable length in comparison between at least two control states with different periods of overlap.

10. Apparatus according to one of the preceding claims, wherein the pneumatic means comprises a pneumatic compressor, wherein the apparatus is adapted to allow the compressor in the activated state to run at different control states with different impact speeds of the projectile (8) at the same rotational frequency, preferably in principle in the activated state to run at always the same rotational frequency.

11. Apparatus according to one of the preceding claims, wherein the projectile (8) can be moved with an impact pulse of between 2 gm/s and 300 gm/s upon impact onto the applicator (6).

12. Apparatus according to one of the preceding claims, adapted to vary, in an iterative operating state with directly successive forward movements of the projectile (8) for impact onto the applicator (6) and return movements, the impact speed and/or the time duration of the combined forward and return movement from one to the next such combined forward and return movement.

13. Apparatus according to one of the preceding claims, having a measuring means for detecting a passage of the projectile (8) at a point of the movement path, which measuring means is coupled to the control means (54).

## Revendications

1. Appareil pour le traitement du corps humain ou animal avec des ondes de pression mécaniques, l'appareil comprenant :
- un projectile (8) guidé dans l'appareil le long d'une trajectoire de déplacement,
- un applicateur (6) à une extrémité de la trajectoire de déplacement,
- un dispositif pneumatique pour appliquer une pression pneumatique au projectile (8) en vue d'un déplacement le long de la trajectoire de déplacement,
le projectile (8) étant conçu pour percuter l'applicateur (6) afin de générer les ondes de pression mécaniques,
le dispositif pneumatique comprenant un dispositif à double soupape (1, 2) pour appliquer une pression pneumatique au projectile (8) en direction de l'applicateur (6) pendant un premier temps de mise en marche et pour appliquer une pression pneumatique au projectile (8) dans le sens inverse pendant un deuxième temps de mise en marche et un dispositif de commande (54) pour commander le dispositif à double soupape (1, 2),
**caractérisé en ce que** l'appareil est conçu pour maintenir un temps d'espacement entre le premier temps de mise en marche et le deuxième temps de mise en marche ou inversement et pour commander une vitesse de percussion du projectile (8) lors de la percussion sur l'applicateur (6) au moyen du temps d'espacement.

2. Appareil selon la revendication 1, dans lequel le dispositif à double soupape (1, 2) comprend une première soupape pour appliquer une pression pneumatique au projectile (8) en direction de l'applicateur (6) et une deuxième soupape pour appliquer une pression pneumatique au projectile (8) dans le sens inverse, lesquelles peuvent être commandées de préférence indépendamment l'une de l'autre par le dispositif de commande (54).

3. Appareil selon la revendication 1, dans lequel le dispositif à double soupape (1, 2) comprend une « soupape combinée » qui, en fonction de la commande par le dispositif de commande (54), adopte un premier état de commutation pour appliquer une pression au projectile (8) en direction de l'applicateur (6) ou un deuxième état de commutation pour appliquer une pression au projectile (8) dans le sens inverse, le raccord pneumatique utilisé dans l'autre état de commutation respectif pour appliquer une pression au projectile (8) étant ventilé par la soupape combinée dans chacun de ces états de commutation.

4. Appareil selon la revendication 2, dans lequel au moins l'une des deux soupapes est une soupape à deux voies qui applique une pression pneumatique à un volume pneumatique entre elle-même et le projectile (8) dans une première position de commutation pendant le temps de mise en marche respectif pour appliquer une pression au projectile (8) et qui ventile ce volume pneumatique dans une deuxième position de commutation.

5. Appareil selon l'une quelconque des revendications précédentes, dans lequel le premier temps de mise en marche est de longueur variable par comparaison entre au moins deux états de commande avec des temps d'espacement différents.

6. Appareil selon l'une quelconque des revendications précédentes, conçu pour, dans une partie d'états de commande, terminer l'un des deux temps de mise en marche seulement après le début de l'autre des deux temps de mise en marche et de préférence terminer l'un des temps de mise en marche pendant l'autre temps de mise en marche, de sorte que le premier et le deuxième temps de mise en marche se chevauchent pendant un temps de chevauchement.

7. Appareil selon la revendication 6, conçu pour, dans la partie des états de commande, commander une vitesse de percussion du projectile (8) lors de la percussion sur l'applicateur (6) au moyen d'une partie du premier temps de mise en marche en dehors du temps de chevauchement qui lui appartient.

8. Appareil selon la revendication 7, dans lequel le plus ancien des deux temps de mise en marche est de longueur constante par comparaison entre au moins deux états de commande avec des périodes de temps différentes du chevauchement entre le premier et le deuxième temps de mise en marche.

9. Appareil selon la revendication 7 ou 8, dans lequel le plus tardif des deux temps de mise en marche est variable par comparaison entre au moins deux états de commande avec des périodes de temps différentes du chevauchement.

10. Appareil selon l'une quelconque des revendications précédentes, dans lequel le dispositif pneumatique comprend un compresseur pneumatique, l'appareil étant conçu pour, dans l'état mis en marche, faire fonctionner le compresseur avec différentes vitesses de percussion du projectile (8) à la même vitesse de rotation dans différents états de commande, de préférence en principe, dans l'état mis en marche, le faire fonctionner toujours à la même vitesse de rotation.

11. Appareil selon l'une quelconque des revendications précédentes, dans lequel le projectile (8) peut être déplacé avec une impulsion de percussion comprise entre 2 gm/s et 300 gm/s lors de la percussion sur l'applicateur (6).

12. Appareil selon l'une quelconque des revendications précédentes, conçu pour, dans un état de fonctionnement itératif avec des mouvements aller et retour directement successifs du projectile (8) pour la percussion sur l'applicateur (6), modifier la vitesse de percussion et/ou la durée du mouvement aller et retour combiné d'un tel mouvement aller et retour combiné au mouvement suivant.

13. Appareil selon l'une quelconque des revendications précédentes, comprenant un dispositif de mesure pour détecter un passage du projectile (8) à un endroit de la trajectoire de déplacement, lequel dispositif de mesure est couplé au dispositif de commande (54).
